# EUROPÄISCHE PATENTSCHRIFT

(11) **EP 4 203 811 B1**
(45) Veröffentlichungstag und Bekanntmachung des Hinweises auf die Patenterteilung: **23.10.2024**
(21) Anmeldenummer: 21777412.4
(22) Anmeldetag: 27.08.2021
(51) Int. Cl.: A61B 17/122, A61B 17/00, A61B 17/128, B33Y 80/00

(54) **MEDIZINISCHER CLIP UND VERFAHREN ZUM HERSTELLEN EINES MEDIZINISCHEN CLIPS**
MEDICAL CLIP AND METHOD FOR MAKING A MEDICAL CLIP
PINCE MÉDICALE ET MÉTHODE POUR FABRIQUER UNE PINCE MÉDICALE

(30) Priorität: 27.08.2020 DE 102020122392
(43) Veröffentlichungstag der Anmeldung: 05.07.2023
(73) Patentinhaber: Aesculap AG, 78532 Tuttlingen (DE)
(72) Erfinder: PLEIL, Thomas, 78073 Bad Dürrheim (DE); SCHOLTEN, Thomas, 78532 Tuttlingen (DE); WILLMANN, Michael, 78048 Villingen-Schwenningen (DE); HAPPLE, Alexander, 78183 Hüfingen (DE); BEGER, Jens, 78532 Tuttlingen (DE)
(74) Vertreter: Hoeger, Stellrecht & Partner Patentanwälte mbB
(86) Internationale Anmeldenummer: PCT/EP2021/073763
(87) Internationale Veröffentlichungsnummer: WO 2022/043506

(56) Entgegenhaltungen:
- EP-B1- 0 724 406
- CN-A- 105 902 292
- CN-A- 105 902 292
- DE-A1- 102017 105 706
- DE-A1- 102018 103 903
- US-A1- 2013 184 726
- ADAM COHEN ET AL: "Microscale metal additive manufacturing of multi-component medical devices", RAPID PROTOTYPING JOURNAL, vol. 16, no. 3, 27 April 2010 (2010-04-27), GB, pages 209 - 215, XP055704003, ISSN: 1355-2546, DOI: 10.1108/13552541011034889

## Beschreibung

Die vorliegende Erfindung betrifft einen medizinischen Clip, insbesondere in Form eines Aneurysmenclips, welcher einen ersten Klemmarm, einen zweiten Klemmarm und ein vorspannendes Element umfasst, wobei der erste Klemmarm mit einem ersten Ende des vorspannenden Elements verbunden ist, wobei der zweite Klemmarm mit einem zweiten Ende des vorspannenden Elements verbunden ist und wobei der erste Klemmarm und der zweite Klemmarm in einer Grundstellung des Clips einander maximal angenähert sind, insbesondere aneinander anliegend, und entgegen der Wirkung des vorspannenden Elements von der Grundstellung in eine Öffnungsstellung voneinander weg bewegbar sind, wobei der medizinische Clip mindestens einen ersten Clipabschnitt und mindestens einen zweiten Clipabschnitt umfasst.

Ferner betrifft die vorliegende Erfindung ein Verfahren zum Herstellen eines medizinischen Clips, insbesondere in Form eines Aneurysmenclips, wobei der Clip mit einem ersten Klemmarm, einem zweiten Klemmarm und einem vorspannenden Element ausgebildet wird derart, dass der erste Klemmarm mit einem ersten Ende des vorspannenden Elements verbunden ist, dass der zweite Klemmarm mit einem zweiten Ende des vorspannenden Elements verbunden ist und dass der erste Klemmarm und der zweite Klemmarm in einer Grundstellung des Clips einander maximal angenähert sind, insbesondere aneinander anliegend, und entgegen der Wirkung des vorspannenden Elements von der Grundstellung in eine Öffnungsstellung voneinander weg bewegbar sind.

Medizinische Clips der eingangs beschriebenen Art sind in vielfältigen Ausführungsformen bekannt. Beispiele sind insbesondere in der DE 10 2018 103 903 A1 beschrieben.

Clips der eingangs beschriebenen Art werden insbesondere in Form von Aneurysmenclips zur neurochirurgischen Behandlung von kranialen Aneurysmen eingesetzt. Bei der Behandlung ist es wichtig, dass der zur Form und Größe des Aneurysmas passende Clip appliziert wird. Daher benötigen insbesondere Neurochirurgen eine große Anzahl unterschiedlicher Aneurysmenclips, die sich in ihrer Größe, in der Form der Klemmarme, auch als Maulteilgeometrie bezeichnet, sowie in der Schließkraft voneinander unterscheiden. Dies führt in der Praxis insbesondere dazu, dass ein Operateur für eine optimale Behandlung eines Aneurysmas aus mehreren hundert verschiedenen medizinischen Clips den jeweils am besten geeigneten Clip auswählt, um ein Aneurysma abzuklemmen.

Die Herstellung derartiger Aneurysmenclips erfordert zahlreiche Prozessschritte. Dies führt zu einer werkzeugintensiven und teuren Herstellung der Clips. In der CN 105 902 292 B sind ein Aneurysmenclip sowie ein Verfahren zur Personalisierung derartiger Clips offenbart. Aus der DE 10 2018 103 903 A1 und der US 2013/0184726 A1 sind chirurgische Clips bekannt. In der DE 10 2017 105 706 A1 sind ein additiv hergestelltes medizintechnisches Instrument sowie ein Verfahren zur Herstellung eines solchen Instruments beschrieben. Ein generatives Herstellungsverfahren für eine medizinische Vorrichtung mit mehreren Komponenten ist in der wissenschaftlichen Veröffentlichung ADAM COHEN ET AL: "Microscale metal additive manufacturing of multi-component medical devices", RAPID PROTOTYPING JOURNAL, Bd. 16, Nr. 3, 27. April 2010 (2010-04-27), Seiten 209-215, XP055704003, GB, ISSN: 1355-2546, DOI: 10.1108/13552541011034889, beschrieben. Und schließlich betrifft die EP 0 724 406 B1 ein Verfahren zum Herstellen eines Aneurysmenclips.

Es ist daher eine Aufgabe der vorliegenden Erfindung, medizinische Clips und Verfahren der eingangs beschriebenen Art zu verbessern.

Diese Aufgabe wird bei einem medizinischen Clip der eingangs beschriebenen Art erfindungsgemäß dadurch gelöst, dass er teilweise durch ein generatives Herstellungsverfahren ausgebildet ist und dass der mindestens eine zweite Clipabschnitt durch ein generatives Herstellungsverfahren direkt an den mindestens einen ersten Clipabschnitt angeformt ist.

Die vorgeschlagene Weiterbildung bekannter medizinischer Clips ermöglicht es insbesondere, unterschiedliche Herstellungsverfahren zur Herstellung des medizinischen Clips zu nutzen. Beispielsweise kann das vorspannende Element, welches insbesondere andere mechanische Eigenschaften als die Klemmarme erfordert, aus einem anderen Material oder durch ein anderes Herstellungsverfahren ausgebildet werden. Den Clip teilweise durch ein generatives Herstellungsverfahren auszubilden, ermöglicht es zudem, nur eine minimale Anzahl von Teilen medizinischer Clips, beispielsweise vorspannender Elemente, bereitzustellen. Klemmarme und weitere Teile des Clips können dann durch ein generatives Herstellungsverfahren stoffschlüssig mit den vorgehaltenen Teilen verbunden werden. So lassen sich insbesondere auf einfache Weise patientenindividuelle Clips ausbilden. Zudem müssen nicht mehr hunderte verschiedener Clips bereitgehalten werden. Durch das generative Herstellungsverfahren kann so die Zahl der bereitzuhaltenden Clips reduziert werden. Ferner können mechanische Eigenschaften unterschiedlicher Bereiche des Clips optimiert werden. Beispielsweise kann das vorspannende Element durch ein generatives Herstellungsverfahren ausgebildet werden. Die Klemmarme können insbesondere direkt durch generative Herstellung an das vorspannende Element angeformt werden. Um insbesondere unterschiedliche Bereiche des Clips mit unterschiedlichen optimierten Eigenschaften auszustatten, ist es vorteilhaft, dass der medizinische Clip mit mindestens einem ersten Clipabschnitt und mindestens einem zweiten Clipabschnitt ausgebildet wird. Durch das direkte Anformen des mindestens einen zweiten Clipabschnitts an den mindestens eine ersten Clipabschnitt kann insbesondere auf Verbindungsverfahren verzichtet werden, um die Clipabschnitte miteinander zu verbinden, die in unerwünschter Weise zu einem Wärmeeintrag in die Clipabschnitte führen können. Insbesondere kann so ein Verbinden der Clipabschnitte durch Schweißen vermieden werden. Durch einen undefinierten Wärmeeintrag kann es insbesondere zu einer Wärmebehandlung des vorspannenden Elements kommen, wodurch sich eine Schließkraft des vorspannenden Elements und damit des medizinischen Clips in undefinierter Weise ändern kann. Direkt Anformen in diesem Sinne bedeutet also insbesondere, dass die Clipabschnitte nicht separat ausgebildet und dann miteinander verbunden werden, beispielsweise durch Schweißen oder Löten, sondern dass der mindestens eine zweite Clipabschnitt an den bereitgestellten mindestens einen ersten Clipabschnitt unmittelbar angeformt ist. Der mindestens eine erste Clipabschnitt kann ebenfalls durch ein generatives Herstellungsverfahren ausgebildet sein. Die Clipabschnitte können aus demselben Werkstoff oder aus unterschiedlichen Werkstoffen ausgebildet sein. Der mindestens eine zweite Clipabschnitt kann insbesondere patientenindividuell ausgebildet und an eine physiologische Situation des Patienten angepasst werden.

Vorzugsweise sind der erste Klemmarm und/oder der zweite Klemmarm durch ein generatives Herstellungsverfahren ausgebildet. Beispielsweise können sie stoffschlüssig an ein vorspannendes Element angeformt werden, welches entweder ebenfalls durch ein generatives Herstellungsverfahren ausgebildet ist oder konventionell in Form einer durch Kaltumformen ausgebildeten Schenkelfeder. Die Klemmarme durch ein generatives Herstellungsverfahren auszubilden ermöglicht insbesondere eine optimale Anpassung an patientenindividuelle Erfordernisse. Letztlich ist ein Operateur dann nicht mehr auf ein konkretes Angebot von medizinischen Clips beschränkt, sondern kann in Abhängigkeit einer Behandlungssituation einen medizinischen Clip mit für den Patienten optimal angepassten Klemmarmen herstellen lassen und applizieren. Ferner haben generativ ausgebildete Klemmarme insbesondere auch den Vorteil, dass sie relativ weich sind. Dies ermöglichst es, sie in ihrer Form und Gestalt noch weiter an individuelle Bedürfnisse eines Patienten anzupassen. Ist der Klemmarm in seine endgültige Form gebracht, kann er durch Härten, beispielsweise in einem weiteren Verfahrensschritt, verfestigt werden, so dass er seine Form dauerhaft behält.

Günstig ist es, wenn das vorspannende Element durch ein generatives Herstellungsverfahren ausgebildet ist. Klemmarme lassen sich an ein solches vorspannendes Element beispielsweise stoffschlüssig durch ein generatives Herstellungsverfahren anfügen. Alternativ ist es auch möglich, an Klemmarme, die durch einen Kaltumformprozess ausgebildet wurden, ein vorspannendes Element direkt durch ein generatives Herstellungsverfahren anzuformen.

Gemäß einer weiteren bevorzugten Ausführungsform der Erfindung kann vorgesehen sein, dass der erste Klemmarm ein dem vorspannenden Element zugewandtes Ende aufweist, dass der zweite Klemmarm ein dem vorspannenden Element zugewandtes Ende aufweist, dass das dem vorspannenden Element zugewandte Ende des ersten Klemmarms über einen ersten Verbindungsabschnitt mit dem ersten Ende des vorspannenden Elements verbunden ist und dass das dem vorspannenden Element zugewandte Ende des zweiten Klemmarms über einen zweiten Verbindungsabschnitt mit dem zweiten Ende des vorspannenden Elements verbunden ist. Diese Ausgestaltung ermöglicht es insbesondere, eine Öffnungsweite des Clips in gewünschter Weise einzustellen. Insbesondere ist es möglich, über die Verbindungsabschnitte einen Abstand der Klemmarme in der Grundstellung vorzugeben. Die Verbindungsabschnitte können entweder einstückig durch einen Kaltumformprozess mit dem vorspannenden Element ausgebildet sein oder durch ein generatives Herstellungsverfahren stoffschlüssig mit dem vorspannenden Element verbunden werden.

Günstig ist es, wenn sich der erste Verbindungsabschnitt und der zweite Verbindungsabschnitt in einem Schlussbereich des Clips kreuzen. Eine solche Ausbildung des Clips ermöglicht es insbesondere, durch Zusammendrücken des vorspannenden Elements, beispielsweise durch Tordieren einer Schenkelfeder, den Clip zu öffnen, also die Klemmarme von der Grundstellung in eine Öffnungsstellung, in welcher sie weiter voneinander entfernt sind als in der Grundstellung, zu bewegen. Ferner kann durch das Kreuzen der Verbindungsabschnitte auch eine Führung der Klemmarme relativ zueinander beim Öffnen und Schließen des Clips vorgegeben werden.

Um insbesondere eine Führung der Klemmarme beim Öffnen und Schließen zu verbessern, ist es vorteilhaft, wenn der Schlussbereich in Form eines Durchsteckschlusses ausgebildet ist. Insbesondere kann so das sogenannte "scissoring" weitgehend vermieden werden. Die Klemmarme können also nicht aneinander vorbeigleiten und dadurch zwischen ihnen eingeklemmtes Gewebe durch Abscheren verletzen.

Ein Durchsteckschluss lässt sich beispielsweise auf einfache Weise dadurch realisieren, dass der eine der zwei Verbindungsabschnitte mit einer Schlussbereichsdurchbrechung ausgebildet ist und dass der andere der zwei Verbindungsabschnitte die Schlussbereichsdurchbrechung durchsetzt. So lassen sich die Klemmarme beim Öffnen und Schließen in definierter Weise führen.

Vorteilhaft ist es, wenn der erste Verbindungsabschnitt und/oder der zweite Verbindungsabschnitt durch spanende Bearbeitung ausgebildet sind. Auf diese Weise können insbesondere eine geforderte Genauigkeit und eine gewünschte Oberflächenqualität im Schlussbereich erreicht werden. Beispielsweise kann einer oder können beide Verbindungsabschnitte einstückig, insbesondere monolithisch mit dem vorspannenden Element ausgebildet werden. An die Verbindungsabschnitte kann dann beispielsweise distalseitig jeweils ein Klemmarm stoffschlüssig durch ein generatives Herstellungsverfahren angeformt werden.

Günstig ist es, wenn der erste Verbindungsabschnitt und/oder der zweite Verbindungsabschnitt durch ein generatives Herstellungsverfahren ausgebildet sind. Wird insbesondere der Verbindungsabschnitt mit der Schlussbereichsdurchbrechung durch ein generatives Herstellungsverfahren ausgebildet, entfällt der sonst übliche Montageaufwand, der mit dem Festlegen eines Schlussplättchens, welches den einseitig geöffnet ausgebildeten Verbindungsabschnitt zur Ausbildung der Schlussbereichsdurchbrechung verschließt, beispielsweise durch Aufschweißen. Dies vereinfacht die Herstellung des medizinischen Clips. Wahlweise kann es dabei günstig sein, wenn einer oder beide Verbindungsabschnitte, die durch ein generatives Herstellungsverfahren ausgebildet sind, durch ein spannendes Verfahren nachgearbeitet werden. Somit kann insbesondere eine Oberflächenqualität der durch ein generatives Herstellungsverfahren ausgebildeten Abschnitte oder Teile des Clips verbessert werden. Sind ein oder beide Verbindungsabschnitte durch ein generatives Herstellungsverfahren ausgebildet, können die Klemmarme unmittelbar an die Verbindungsabschnitte anschließend durch ein generatives Herstellungsverfahren angeformt werden.

Gemäß einer weiteren bevorzugten Ausführungsform der Erfindung kann vorgesehen sein, dass der erste Klemmarm eine erste Klemmfläche aufweist, dass der zweite Klemmarm eine zweite Klemmfläche aufweist und dass in der Grundstellung die erste Klemmfläche und die zweite Klemmfläche aneinander anliegen oder im Wesentlichen aneinander anliegen. Auf diese Weise kann eine definierte Grundstellung für den medizinischen Clip vorgegeben werden.

Vorteilhaft ist es, wenn der erste Klemmarm und der zweite Klemmarm in der Grundstellung gegeneinander vorgespannt sind. Auf diese Weise kann insbesondere eine definierte Schließkraft für den medizinischen Clip vorgegeben werden, beispielsweise durch das vorspannende Element, das die beiden Klemmarme in der Grundstellung gegeneinander vorspannt.

Auf einfache Weise lässt sich der medizinische Clip ausbilden, wenn das vorspannende Element in Form einer Schraubenfeder mit mindestens einer vollständigen Wicklung ausgebildet ist. Die Schraubenfeder kann jedoch auch zwei, drei oder mehr vollständige Wicklungen umfassen.

Um ein vorspannendes Element mit einer hohen Festigkeit zu erhalten, ist es günstig, wenn das vorspannende Element durch einen Kaltumformprozess ausgebildet ist.

Auf einfache Weise lässt sich das vorspannende Element aus einem Draht ausbilden, insbesondere durch Aufwickeln zu einer Schraubenfeder.

Um Abstoßungsreaktionen beim Einsetzen eines medizinischen Clips in einen menschlichen oder tierischen Körper zu vermeiden oder zumindest zu minimieren, ist es günstig, wenn der Clip aus mindestens einem körperverträglichen Werkstoff ausgebildet ist. Insbesondere kann er nur aus einem einzigen körperverträglichen Werkstoff ausgebildet sein. Wenn der Clip nur aus einem einzigen körperverträglichen Werkstoff ausgebildet ist, kann insbesondere ein Anformen von Teilen, beispielsweise Klemmarmen, an Verbindungsabschnitte oder ein vorspannendes Element des Clips, die aus demselben Werkstoff, jedoch aus einem Vollmaterial, ausgebildet sind, auf einfache Weise realisiert werden. Unterschiedliche körperverträgliche Werkstoffe haben insbesondere den Vorteil, dass unterschiedliche Bereiche oder Abschnitte des Clips in Abhängigkeit ihrer Funktion gewählt werden können, um insbesondere ein vorspannendes Element mit hoher Festigkeit einerseits und Klemmarme, die gewebeschonend sind, andererseits, auszubilden.

Vorzugsweise ist der körperverträgliche Werkstoff ein Metall oder ein Kunststoff. Das Metall kann beispielsweise Titan sein. Als Kunststoff kann beispielsweise Polyetheretherketon (PEEK) zum Einsatz kommen.

Günstig ist es, wenn der mindestens eine erste Clipabschnitt durch einen Kaltumformprozess oder durch ein generatives Herstellungsverfahren ausgebildet ist. Kaltumformen ist insbesondere vorteilhaft, wenn der Clipabschnitt eine besonders hohe Festigkeitseigenschaften aufweisen soll. Ein solcher Clipabschnitt kann dann mit einem zweiten Clipabschnitt kombiniert werden, dessen Form durch generative Herstellungsverfahren auf einfache Weise realisiert werden können. Zur Erhöhung der Festigkeit eines generativ hergestellten Clipabschnitts kann insbesondere auch das heißisostatische Pressen (HIP-Prozess) zur Anwendung kommen.

Gemäß einer weiteren bevorzugten Ausführungsform der Erfindung kann vorgesehen sein, dass ein Übergang des mindestens einen ersten Clipabschnitt zum mindestens zweiten Clipabschnitt einen Übergangsbereich definiert. Beispielsweise kann ein Übergangsbereich definiert werden zwischen dem vorspannenden Element und einem Verbindungsabschnitt oder zwischen einem Verbindungsabschnitt und einem Klemmarm. Insbesondere können die beiden Clipabschnitte, die über den Übergangsbereich miteinander verbunden sind, einerseits durch ein Kaltumformverfahren und/oder durch spanende Bearbeitung ausgebildet sein und der andere Clipabschnitt durch ein generatives Herstellungsverfahren. Im Übergangsbereich können somit unterschiedliche Herstellungsverfahren aufeinandertreffen. Der Übergangsbereich ist insbesondere ein Verbindungsbereich zwischen zwei Clipabschnitten, welcher sich über eine endliche Länge, die unter Umständen sehr klein sein kann, erstreckt. Im Übergangsbereich treffen also beispielsweise zwei unterschiedliche Werkstoffe oder Werkstoffe unterschiedlicher Formen aufeinander. Insbesondere ist eine stoffschlüssige Verbindung zwischen zwei Clipabschnitten im Übergangsbereich realisiert.

Vorteilhaft ist es, wenn der Übergangsbereich eine Übergangsbereichslängsachse definiert und sich zwischen einem ersten Übergangsbereichsende und einem zweiten Übergangsbereichsende erstreckt, wenn zwischen dem ersten Übergangsbereichsende und dem Übergangsbereichsende ein Querschnitt quer zur Übergangsbereichslängsachse teilweise durch den mindestens einen ersten Clipabschnitt und teilweise durch den mindestens einen zweiten Clipabschnitt definiert ist. Wird mit anderen Worten der medizinische Clip im Übergangsbereich durchtrennt, werden im Querschnitt Bereiche sichtbar, die durch den mindestens einen ersten Clipabschnitt einerseits und durch den mindestens einen zweiten Clipabschnitt andererseits gebildet sind. Im Übergangsbereich zwischen den beiden Übergangsbereichsenden wird der Clip somit durch die beiden stoffschlüssig miteinander verbundenen Clipabschnitte gebildet.

Vorteilhaft ist es, wenn am ersten Übergangsbereichsende ein Querschnitt quer zur Übergangsbereichslängsachse vollständig durch den mindestens einen ersten Clipabschnitt definiert ist und wenn am zweiten Übergangsbereichsende ein Querschnitt quer zur Übergangsbereichslängsachse vollständig durch den mindestens einen zweiten Clipabschnitt definiert ist. Auf diese Weise können die Übergangsbereichsenden eindeutig definiert werden, da Querschnitte quer zur Übergangsbereichslängsachse jeweils nur durch einen der beiden Clipabschnitte definiert sind. Auf diese Weise kann auch eine Länge des Übergangsbereichs eindeutig definiert werden, nämlich als ein Abstand zwischen den beiden Übergangsbereichsenden.

Günstig ist es, wenn der Übergangsbereich eine Kontaktfläche zwischen dem mindestens einen ersten Clipabschnitt und dem mindestens einen zweiten Clipabschnitt definiert und wenn die Kontaktfläche mindestens einer Querschnittsfläche des Clips im Übergangsbereich quer zur Übergangsbereichslängsachse entspricht. Die Kontaktfläche kann also der Querschnittsfläche des Clips im Übergangsbereich entsprechen oder größer sein. Endet beispielsweise der erste Clipabschnitt mit einer Endfläche quer zur Übergangsbereichslängsachse, wird so die Kontaktfläche definiert. Der zweite Clipabschnitt kann bei dieser Ausgestaltung flächig an die Endfläche des ersten Clipabschnitts anschließen. Eine in Richtung auf den ersten Clipabschnitt hin weisende Endfläche des zweiten Clipabschnitts, die mit dem ersten Clipabschnitt dann beispielsweise stoffschlüssig verbunden ist, weist dann denselben Querschnitt und damit dieselbe Querschnittsfläche auf wie die Endfläche des ersten Clipabschnitts.

Um eine Verbindung zwischen den Clipabschnitten zu verbessern, ist es vorteilhaft, wenn die Kontaktfläche mindestens 50% größer ist als eine Querschnittsfläche des Clips im Übergangsbereich quer zur Übergangsbereichslängsachse. Insbesondere kann die Kontaktfläche mindestens 100% größer sein. Je größer die Kontaktfläche im Verhältnis zur Querschnittsfläche des Clips im Übergangsbereich ist, umso besser eine Verbindung zwischen den beiden Clipabschnitten. Beispielsweise kann die Kontaktfläche auf einfache Weise vergrößert werden, wenn im Übergangsbereich Hinterschneidungen am ersten und/oder zweiten Clipabschnitt ausgebildet sind.

Vorteilhaft ist es, wenn sich der Übergangsbereich über eine Länge erstreckt, welche mindestens etwa 50% eines Durchmessers des Übergangsbereichs entspricht. Insbesondere kann die Länge mindestens etwa 100% eines Durchmessers des Übergangsbereichs entsprechen. Eine Länge des Übergangsbereichs wie angegeben vorzusehen, ermöglicht insbesondere eine verbesserte Verbindung zwischen den beiden Clipabschnitten im Übergangsbereich. Insbesondere kann sich durch Vergrößerung der Länge des Übergangsbereichs auch eine Größe der Kontaktfläche vergrößern.

Günstig ist es, wenn an einem der Clipabschnitte ein sich in Richtung auf den mindestens einen anderen Clipabschnitt hin weisender Verbindungsvorsprung ausgebildet ist und wenn am mindestens einen anderen Clipabschnitt eine Verbindungsvorsprungsaufnahme ausgebildet ist, die den Verbindungsvorsprung kraft- und/oder form- und/oder stoffschlüssig aufnimmt. Diese Ausgestaltung ermöglicht es insbesondere, eine stoffschlüssige Verbindung zwischen den beiden Clipabschnitten zusätzlich zu stabilisieren. Insbesondere dann, wenn einer der Clipabschnitte durch ein Kaltumformverfahren ausgebildet ist, kann so eine dauerhafte und sichere Verbindung zwischen den Clipabschnitten erreicht werden, beispielsweise auch dann, wenn einer der Clipabschnitte durch ein generatives Herstellungsverfahren ausgebildet ist.

Um eine Verbindung der Clipabschnitte weiter zu verbessern, ist es günstig, wenn am Verbindungsvorsprung und/oder an der Verbindungsvorsprungsaufnahme mindestens eine Hinterschneidung ausgebildet ist. So lässt sich insbesondere eine sichere formschlüssige Verbindung zwischen den Clipabschnitten erreichen, und zwar unabhängig davon, ob diese zusätzlich stoffschlüssig miteinander verbunden sind oder nicht.

Auf einfache Weise lässt sich eine formschlüssige Verbindung ausbilden, wenn die Hinterschneidung in einer Richtung quer, insbesondere senkrecht, bezogen auf die Übergangsbereichslängsachse geöffnet ist. Insbesondere an einem durch ein Kaltumformverfahren ausgebildeten Clipabschnitt lassen sich so Hinterschneidungen auf einfache Weise realisieren, beispielsweise durch eine spanende Bearbeitung.

Eine gute und sichere Verbindung der beiden Clipabschnitte miteinander im Übergangsbereich kann insbesondere dadurch erzielt werden, dass die Hinterschneidung in Form einer Nut ausgebildet ist. Insbesondere kann die Nut in Form einer Ringnut ausgebildet sein, beispielsweise in Form einer umlaufenden Ringnut. So kann ein allseitiges Ineinandergreifen der Clipabschnitte im Übergangsbereich erreicht werden. Insbesondere kann so ein einfaches Abziehen der Clipabschnitte voneinander auf wirkungsvolle Weise verhindert werden.

Um die Herstellung des medizinischen Clips zu vereinfachen, ist es günstig, wenn der Verbindungsvorsprung und/oder die Verbindungsvorsprungsaufnahme rotationssymmetrisch ausgebildet sind.

Vorzugsweise definiert der Verbindungsvorsprung ein Vorsprungsende, welches spitz oder abgerundet ausgebildet ist. So lässt sich auf einfache Weise eine Kontaktfläche zwischen den beiden Clipabschnitten vergrößern.

Vorteilhaft ist es, wenn die Kontaktfläche mindestens teilweise, insbesondere vollständig, quer zur Übergangsbereichslängsachse verläuft. Sie kann insbesondere auch parallel zur Übergangsbereichslängsachse verlaufen. Quer zur Übergangsbereichslängsachse ist insbesondere vorteilhaft, um beispielsweise Hinterschneidungen zu realisieren.

Günstig ist es, wenn ein Neigungswinkel zwischen der Übergangsbereichslängsachse und der Kontaktfläche in einem Bereich von etwa 35° bis etwa 55° liegt. Insbesondere kann er etwa 45° betragen. Die Kontaktfläche mit einem Neigungswinkel im angegebenen Bereich relativ zur Übergangsbereichslängsachse zu neigen ermöglicht auf einfache Weise eine Vergrößerung der Kontaktfläche und damit eine Verbesserung der Verbindung zwischen den Clipabschnitten.

Vorzugsweise ist die Kontaktfläche eben oder gekrümmt. Beispielsweise kann die Kontaktfläche vom Clipabschnitt, der nicht durch ein generatives Fertigungsverfahren ausgebildet ist, weg weisend konvex gekrümmt sein. Ein solches Ende des Clipabschnitts kann insbesondere auf einfache Weise beispielsweise durch spanende Bearbeitung oder durch Kaltumformen realisiert werden.

Bestimmte Bereiche des medizinischen Clips in gewünschter Weise Formen zu können, ist auf einfache Weise insbesondere dadurch realisierbar, dass das generative Herstellungsverfahren Laser-Auftragsschweißen oder ein thermisches Spritzverfahren, insbesondere ein Metall-Pulver-Auftragsverfahren (MPA), ist. Mit insbesondere diesen Verfahren können Bereiche des Clips oder Clipabschnitte in gewünschter Weise geformt werden, insbesondere patientenindividuell.

Gemäß einer weiteren bevorzugten Ausführungsform der Erfindung kann vorgesehen sein, insbesondere auch bei einem medizinischen Clip der eingangs beschriebenen Art, dass das vorspannende Element in Form einer Schraubenfeder mit mindestens einer vollständigen Windung ausgebildet ist und dass am vorspannenden Element im Bereich des ersten Endes und/oder des zweiten Endes eine Windungsaufnahme ausgebildet ist und dass die Windungsaufnahme in Richtung auf die benachbarte Windung weisend ausgebildet ist. Die Windungsaufnahme vergrößert insbesondere einen Abstand zwischen einer äußeren Oberfläche der Windungsaufnahme und einer äußeren Oberfläche der der Windungsaufnahme gegenüberliegenden Windung der Schraubenfeder. So kann beim Tordieren der Schraubenfeder vollständig oder im Wesentlichen vollständig verhindert werden, dass das erste Ende mit der Windung der Schraubenfeder in Kontakt tritt, beispielsweise wenn der Clip geöffnet wird.

Oder mit anderen Worten ausgedrückt kann durch eine oder zwei Windungsaufnahmen in der beschriebenen Weise eine Kollision der Schraubenfeder mit insbesondere dem Verbindungsabschnitt verhindert werden. Dadurch lässt sich eine Reibung im Bereich der Schraubenfeder beim Öffnen des Clips vermeiden oder weitestgehend vermeiden, was eine genauere Einstellung der Schließkräfte des Clips ermöglicht.

Auf einfache Weise lässt sich die Windungsaufnahme durch eine Abflachung oder eine flache Ausnehmung am vorspannenden Element ausbilden. Eine flache Ausnehmung kann insbesondere in Richtung auf die benachbarte Windung hin weisend konkav gekrümmt sein. Eine solche Windungsaufnahme kann insbesondere bei einem Rohling, aus dem das vorspannende Element ausgebildet ist, auf einfache Weise durch eine spanende Bearbeitung ausgebildet werden.

Günstiger Weise ist der Clip in Form eines Aneurysmenclips ausgebildet. Dies ermöglicht den Einsatz des Clips zur Behandlung von Aneurysmen, insbesondere innerhalb des Schädels eines Patienten.

Die eingangs gestellte Aufgabe wird ferner bei einem Verfahren der eingangs beschriebenen Art erfindungsgemäß dadurch gelöst, dass der medizinische Clip teilweise durch ein generatives Herstellungsverfahren ausgebildet wird und dass der mindestens eine zweite Clipabschnitt durch ein generatives Herstellungsverfahren direkt an den mindestens einen ersten Clipabschnitt angeformt wird.

Wie bereits oben eingehend dargelegt, kann so auf eine kostengünstige Weise eine große Variantenvielfalt medizinischer Clips hergestellt werden. Beispielsweise können vorspannende Elemente, die eine Schließkraft des Clips definieren, in einer endlichen Anzahl bereitgestellt werden. Klemmarme, die direkt oder indirekt mit dem vorspannenden Element verbunden sind, beispielsweise indirekt über Verbindungsabschnitte, können dann patientenindividuell für eine optimale Behandlung, beispielsweise von Aneurysmen, geformt werden. Dadurch, dass der mindestens eine zweite Clipabschnitt durch ein generatives Herstellungsverfahren direkt an den mindestens einen ersten Clipabschnitt angeformt wird, kann insbesondere auf Verbindungsverfahren verzichtet werden, um die Clipabschnitte miteinander zu verbinden, die in unerwünschter Weise zu einem Wärmeeintrag in die Clipabschnitte führen können. Insbesondere kann so ein Verbinden der Clipabschnitte durch Schweißen vermieden werden. Durch einen solchen Wärmeeintrag kann es beispielsweise zu einer undefinierten Wärmebehandlung insbesondere des vorspannenden Elements kommen, wodurch sich eine Schließkraft des vorspannenden Elements und damit des medizinischen Clips in unbekannter und undefinierter Weise ändern kann. Direkt Anformen in diesem Sinne bedeutet also insbesondere, dass die Clipabschnitte nicht separat ausgebildet und dann miteinander verbunden werden, beispielsweise durch Schweißen oder Löten, sondern dass der mindestens eine zweite Clipabschnitt an den bereitgestellten mindestens einen ersten Clipabschnitt unmittelbar angeformt ist. Beispielsweise können ein Klemmarm oder ein Verbindungsabschnitt einen zweiten Clipabschnitt in diesem Sinne bilden.

Diese können durch das generative Herstellungsverfahren auf einfache Weise patientenindividuell geformt werden.

Eine patientenindividuelle Formgebung des Clips kann insbesondere auf einfache Weise dadurch realisiert werden, dass der erste Klemmarm und/oder der zweite Klemmarm durch ein generatives Herstellungsverfahren ausgebildet werden.

Ferner kann es vorteilhaft sein, wenn das vorspannende Element durch ein generatives Herstellungsverfahren ausgebildet wird. So kann beispielsweise eine Form des vorspannenden Elements ebenfalls patientenindividuell ausgebildet werden. An ein derart ausgebildetes vorspannendes Element können Klemmarme ebenfalls durch ein generatives Herstellungsverfahren angeformt werden. Alternativ können Klemmarme auch durch ein Kaltumformverfahren ausgebildet und das vorspannende Element direkt durch ein generatives Herstellungsverfahren angeformt werden.

Gemäß einer bevorzugten Ausführungsform des Verfahrens kann vorgesehen sein, dass der der erste Klemmarm ein dem vorspannenden Element zugewandtes Ende aufweist, dass der zweite Klemmarm ein dem vorspannenden Element zugewandtes Ende aufweist, dass das dem vorspannenden Element zugewandte Ende des ersten Klemmarms über einen ersten Verbindungsabschnitt mit dem ersten Ende des vorspannenden Elements verbunden wird und dass das dem vorspannenden Element zugewandte Ende des zweiten Klemmarms über einen zweiten Verbindungsabschnitt mit dem zweiten Ende des vorspannenden Elements verbunden wird. Die Verbindungsabschnitte vorzusehen ermöglicht insbesondere eine Verbindung von standardisiert bereitgestellten Klemmarmen einerseits und vorspannenden Elementen andererseits. Beispielsweise können sowohl die Klemmarme als auch das vorspannende Element durch ein Kaltumformverfahren ausgebildet werden. Eine Verbindung kann dann beispielsweise durch die Verbindungsabschnitte realisiert werden, die einerseits stoffschlüssig mit dem vorspannenden Element und andererseits stoffschlüssig mit einem Klemmarmen verbunden werden. Durch Form und Länge der Verbindungsabschnitte kann insbesondere eine Öffnungsweite des medizinischen Clips vorgegeben werden. Zudem ermöglichen die Verbindungsabschnitte auch eine Führung des Clips beim Öffnen und Schließen.

Vorteilhafterweise werden der erste Verbindungsabschnitt und der zweite Verbindungsabschnitt in einem Schlussbereich des Clips sich kreuzend ausgebildet. Kreuzende Verbindungsabschnitte können insbesondere aneinander anliegen oder mit wenig Spiel ein seitliches Ausweichen relativ zueinander verhindern, beispielsweise beim Öffnen und Schließen des Clips.

Insbesondere das oben beschriebene Scissoring bei medizinischen Clips kann auf einfache Weise insbesondere dadurch verhindert werden, dass der Schlussbereich in Form eines Durchsteckschlusses ausgebildet wird.

Günstig ist es, wenn der eine der zwei Verbindungsabschnitte mit einer Schlussbereichsdurchbrechung ausgebildet wird und wenn der andere der zwei Verbindungsabschnitte die Schlussbereichsdurchbrechung durchsetzend ausgebildet wird. Dies ermöglicht es insbesondere, den einen Verbindungsabschnitt in der Schlussbereichsdurchbrechung beim Öffnen und Schließen des Clips zu führen. Insbesondere kann die Schlussbereichsdurchbrechung in Form eines Langlochs ausgebildet sein. Diese kann dann insbesondere auch Anschläge für eine maximale Öffnung des Clips und/oder für eine Grundstellung des Clips, in der die Klemmarme einander maximal angenähert sind, insbesondere aneinander anliegen, definieren.

Insbesondere mit hoher Präzision und einer gewünschten Oberflächenqualität können die Verbindungsabschnitte ausgebildet werden, wenn der erste Verbindungsabschnitt und/oder der zweite Verbindungsabschnitt durch spanende Bearbeitung ausgebildet werden.

Die Herstellung des medizinischen Clips kann insbesondere dadurch vereinfacht werden, dass der erste Verbindungsabschnitt und/oder der zweite Verbindungsabschnitt durch ein generatives Herstellungsverfahren ausgebildet werden. Weist beispielsweise einer der zwei Verbindungsabschnitte eine Schlussbereichsdurchbrechung auf, so kann diese durch ein generatives Herstellungsverfahren in einem Arbeitsschritt ausgebildet werden. Wird der Schlussbereich im Gegensatz hierzu durch eine spanende Bearbeitung ausgebildet, wird am einen Verbindungsabschnitt eine Ausnehmung ausgebildet, die nach Einführen des anderen Verbindungsabschnitts mit einem Verschlusselement, beispielsweise einem Verschlussplättchen, verschlossen werden muss. Derartige Verschlussplättchen sind sehr klein und müssen in einem zusätzlichen Arbeitsschritt, beispielsweise durch Schweißen, am Verbindungsabschnitt festgelegt werden. Dieser Fertigungsaufwand kann durch generative Ausbildung mindestens des einen Verbindungsabschnitts vereinfacht werden.

Vorteilhaft ist es, wenn am ersten Klemmarm eine erste Klemmfläche ausgebildet wird, wenn am zweiten Klemmarm eine zweite Klemmfläche ausgebildet wird und wenn in der Grundstellung die erste Klemmfläche und die zweite Klemmfläche aneinander anliegend oder im Wesentlichen aneinander anliegend ausgebildet werden. Dies ermöglicht insbesondere die Ausbildung medizinischer Clips, die in einer Grundstellung geschlossen sind.

Günstig ist es, wenn der erste Klemmarm und der zweite Klemmarm in der Grundstellung gegeneinander vorgespannt werden. Auf diese Weise kann insbesondere eine minimale Schließkraft des medizinischen Clips vorgegeben werden.

Auf einfache Weise lässt sich der Clip ausbilden, wenn das vorspannende Element in Form einer Schraubenfeder mit mindestens einer vollständigen Windung ausgebildet wird. Die Schraubenfeder kann insbesondere im Uhrzeigersinn oder gegen den Uhrzeigersinn gewickelt werden. Insbesondere lässt sich so der medizinische Clip kompakt ausbilden. Zudem ist er beispielsweise mit bildgebenden Verfahren, insbesondere durch Röntgen, auf einfache Weise detektierbar, beispielsweise um seine genaue Position und Orientierung im Körper eines Patienten festzustellen.

Günstigerweise wird das vorspannende Element durch einen Kaltumformprozess ausgebildet. So kann insbesondere eine sehr hohe Festigkeit des Clips im Bereich des vorspannenden Elements erreicht werden.

Auf einfache Weise lässt sich das vorspannende Element aus einem Draht ausbilden. Beispielsweise kann der Draht zu einer Schraubenfeder gewickelt werden mit einer, zwei oder noch mehr Windungen.

Um Abstoßungsreaktionen zu vermeiden, ist es günstig, wenn der Clip aus mindestens einem körperverträglichen Werkstoff, insbesondere nur aus einem einzigen körperverträglichen Werkstoff, ausgebildet wird.

Vorteilhafterweise wird der mindestens eine erste Clipabschnitt durch einen Kaltumformprozess oder durch ein generatives Herstellungsverfahren ausgebildet. Durch einen Kaltumformprozess können insbesondere Clipabschnitte mit hoher Festigkeit realisiert werden. Erste Clipabschnitte mit einer gewünschten Form, insbesondere patientenindividuell, können auf einfache Weise durch ein generatives Herstellungsverfahren ausgebildet werden.

Günstig ist es, wenn der mindestens eine erste Clipabschnitt in Form des vorspannenden Elements ausgebildet wird und wenn das vorspannende Element beim Anformen des zweiten Clipabschnitts unter Vorspannung gehalten wird. Unter Vorspannung halten kann insbesondere durch Tordieren des vorspannenden Elements erfolgen, wenn dieses durch eine Schraubenfeder gebildet ist. Diese Vorgehensweise hat insbesondere den Vorteil, dass an das vorspannende Element ein oder zwei weitere Clipabschnitte durch ein generatives Herstellungsverfahren angeformt, beispielsweise an das vorspannende Element, werden können, ohne sich gegenseitig zu stören. Auf diese Weise kann insbesondere erreicht werden, dass der medizinische Clip in einer Grundstellung, in der die Klemmarme aneinander anliegen, die Klemmarme gegeneinander vorgespannt sind. Wird das vorspannende Element beim Anformen zweiter Clipabschnitte nicht unter Vorspannung gehalten, kann allenfalls eine Grundstellung des Clips realisiert werden, in der Klemmarme desselben aneinander anliegen, jedoch ohne gegeneinander vorgespannt zu sein.

Vorteilhaft ist es, wenn ein Übergang des mindestens einen ersten Clipabschnitts zum mindestens einen zweiten Clipabschnitt ausgebildet wird, welcher einen Übergangsbereich definiert. Im Übergangsbereich können also insbesondere zwei Clipabschnitte in gewünschter Weise direkt aneinander angeformt werden, indem einer der Clipabschnitte an den anderen durch ein generatives Herstellungsverfahren angeformt wird. Insbesondere können beide Clipabschnitte durch ein generatives Herstellungsverfahren ausgebildet werden.

Vorteilhaft ist es, wenn der Übergangsbereich eine Übergangsbereichslängsachse definiert und sich zwischen einem ersten Übergangsbereichsende und einem zweiten Übergangsbereichsende erstreckend ausgebildet wird, wenn zwischen dem ersten Übergangsbereichsende und dem zweiten Übergangsbereichsende ein Querschnitt quer zur Übergangsbereichslängsachse derart ausgebildet wird, dass der Querschnitt teilweise durch den mindestens einen ersten Clipabschnitt und den mindestens einen zweiten Clipabschnitt definiert ist. Einen Übergangsbereich zwischen zwei Clipabschnitten in der beschriebenen Weise auszubilden, erhöht insbesondere eine Stabilität der Verbindung. Eine Größe der Kontaktfläche, wie sie oben beschrieben wurde, wird auf diese Weise vergrößert, sodass eine großflächigere stoffschlüssige Verbindung realisierbar ist.

Günstig ist es, wenn der Übergangsbereich derart ausgebildet wird, dass am ersten Übergangsbereichsende ein Querschnitt quer zur Übergangsbereichslängsachse vollständig durch den mindestens einen ersten Clipabschnitt definiert ist und wenn am zweiten Übergangsbereichsende ein Querschnitt quer zur Übergangsbereichslängsachse vollständig durch den mindestens einen zweiten Clipabschnitt definiert ist. In der beschriebenen Weise kann der Übergangsbereich in gewünschter Weise parametrisiert werden, beispielsweise durch eine Länge des Übergangsbereichs, also eine Übergangsbereichslänge, die einem Abstand zwischen dem ersten und dem zweiten Übergangsbereichsende entspricht.

Vorteilhaft ist es, wenn der Übergangsbereich derart ausgebildet wird, dass er eine Kontaktfläche zwischen dem mindestens einen ersten Clipabschnitt und dem mindestens einen zweiten Clipabschnitt definiert und wenn die Kontaktfläche mindestens einer Querschnittsfläche des Clips im Übergangsbereich quer zur Übergangsbereichslängsachse entspricht. Die Kontaktfläche kann also gleich oder größer sein als die Querschnittsfläche des Clips im Übergangsbereich quer zur Übergangsbereichslängsachse. Je größer die Kontaktfläche ist, umso größer sind die aneinander anliegenden Flächenbereiche der beiden Clipabschnitte. Mit größerer Kontaktfläche wird also eine Verbindung, insbesondere eine stoffschlüssige Verbindung, zwischen den Clipabschnitten verbessert.

Vorteilhafterweise wird die Kontaktfläche mindestens 50% größer ausgebildet als eine Querschnittsfläche des Clips im Übergangsbereich quer zur Übergangsbereichslängsachse. Insbesondere kann die Kontaktfläche mindestens 100% größer ausgebildet werden als die Querschnittsfläche des Clips im Übergangsbereich quer zur Übergangsbereichslängsachse. Auf diese Weise lässt sich eine Stabilität der Verbindung der Clipabschnitte auf einfache Weise verbessern.

Günstig ist es, wenn sich der Übergangsbereich über eine Länge erstreckend ausgebildet wird, welche mindestens etwa 50% eines Durchmessers des Übergangsbereichs entspricht, insbesondere mindestens etwa 100%. Einen Übergangsbereich mit einer Länge im angegebenen Bereich auszubilden kann insbesondere eine Stabilität der Verbindung zwischen den Clipabschnitten verbessern. Insbesondere kann eine Länge des Übergangsbereichs auch dazu genutzt werden, die Kontaktfläche zwischen den Clipabschnitten zu vergrößern.

Ferner ist es vorteilhaft, wenn an einem der Clipabschnitte ein sich in Richtung auf den mindestens einen anderen Clipabschnitt hin weisender Verbindungsvorsprung ausgebildet wird und wenn am mindestens einen anderen Clipabschnitt eine Verbindungsvorsprungsaufnahme ausgebildet wird, die den Verbindungsvorsprung kraft- und/oder formschlüssig aufnimmt. So kann eine besonders stabile Verbindung zwischen den Clipabschnitten realisiert werden. Insbesondere ist es vorteilhaft, wenn die Verbindungsvorsprungsaufnahme durch ein generatives Herstellungsverfahren oder Fertigungsverfahren ausgebildet wird. So kann beispielsweise eine formschlüssige Ausnehmung um den Vorsprung herum ausgebildet werden. Insbesondere können so auch auf einfache Weise Hinterschnitte realisiert werden.

Vorzugsweise wird am Verbindungsvorsprung und/oder an der Verbindungsvorsprungsaufnahme mindestens eine Hinterschneidung ausgebildet. Durch eine Hinterschneidung kann insbesondere ein Formschluss realisiert werden, welcher zu einer Verbesserung einer Stabilität der Verbindung zwischen den zwei Clipabschnitten führt.

Auf einfache Weise lässt sich das Verfahren realisieren, wenn die Hinterschneidung in einer Richtung quer, insbesondere senkrecht, bezogen auf die Übergangsbereichslängsachse geöffnet ausgebildet wird. Beispielsweise kann eine solche Hinterschneidung durch spanende Bearbeitung an einem der beiden Clipabschnitte realisiert werden.

Einfach und kostengünstig lässt sich eine Hinterschneidung in Form einer Nut, insbesondere in Form einer Ringnut, ausbilden.

Vorteilhaft ist es, wenn der Verbindungsvorsprung und/oder die Verbindungsvorsprungsaufnahme rotationssymmetrisch ausgebildet werden. Auf diese Weise lässt sich die Herstellung des medizinischen Clips vereinfachen.

Günstig ist es, wenn die Kontaktfläche mindestens teilweise, insbesondere vollständig, quer zur Übergangsbereichslängsachse verläuft. Eine solche Ausgestaltung ermöglicht es insbesondere, die Clipabschnitte mit ebenen Endflächen stumpf miteinander stoffschlüssig zu verbinden, beispielsweise durch direktes generative Anformen. Verläuft die Kontaktfläche nur teilweise, insbesondere auch in mehreren Abschnitten, quer zur Übergangsbereichslängsachse, kann so auf einfache Weise eine Größe der Kontaktfläche in gewünschter Weise eingestellt werden, um eine dauerhafte und stabile Verbindung zwischen den beiden Clipabschnitten ausbilden zu können.

Vorzugsweise wird die Kontaktfläche derart ausgebildet wird, dass ein Neigungswinkel zwischen der Übergangsbereichslängsachse und der Kontaktfläche in einem Bereich von etwa 35° bis etwa 55° liegt, insbesondere etwa 45° beträgt. So kann eine Kontaktfläche auf einfache Weise und mit einer gewünschten Größe ausgebildet werden.

Die Herstellung des medizinischen Clips kann insbesondere dadurch vereinfacht werden, dass die Kontaktfläche eben oder gekrümmt ausgebildet wird.

Vorteilhaft ist es, wenn der Verbindungsvorsprung ein Vorsprungsende definierend ausgebildet wird und wenn das Vorsprungsende spitz oder abgerundet ausgebildet wird. Einen Verbindungsvorsprung mit einem solchen Vorsprungsende auszubilden ermöglicht es auf einfache Weise, eine Kontaktfläche mit einer gewünschten Größe auszubilden.

Gemäß einer bevorzugten Ausführungsform der Erfindung kann vorgesehen sein, dass als generatives Herstellungsverfahren Laser-Auftragsschweißen oder ein thermisches Spritzverfahren, insbesondere ein Metall-Pulver-Auftragsverfahren (MPA), angewandt wird. Beispielsweise kann mit einem solchen generativen Herstellungsverfahren ein Clipabschnitt an einen anderen Clipabschnitt in gewünschter Weise angeformt werden, und zwar unabhängig davon, ob der erste Clipabschnitt ebenfalls durch ein generatives Herstellungsverfahren ausgebildet wurde oder durch ein Kaltumformverfahren.

Ferner kann es vorteilhaft sein, insbesondere auch bei einem Verfahren der eingangs beschriebenen Art, wenn das vorspannende Element in Form einer Schraubenfeder mit mindestens einer vollständigen Windung ausgebildet wird, wenn am vorspannenden Element im Bereich des ersten Endes und/oder des zweiten Endes eine Windungsaufnahme ausgebildet wird und wenn die Windungsaufnahme in Richtung auf die benachbarte Windung weisend ausgebildet wird. Auf diese Weise kann eine Reibung im Bereich der Schraubenfeder, zu der es beim Tordieren derselben kommen kann, wenn die ersten und zweiten Enden beim Zusammenziehen der Schraubenfedern infolge einer Torsion mit der benachbarten Windung in Kontakt kommen, signifikant reduziert werden.

Auf einfache Weise lässt sich der Clip ausbilden, wenn die Windungsaufnahme durch eine Abflachung oder eine flache Ausnehmung am vorspannenden Element ausgebildet wird.

Um den medizinischen Clip insbesondere zur Behandlung kranialer Aneurysmen einsetzen zu können, ist es günstig, wenn der Clip in Form eines Aneurysmenclips ausgebildet wird.

Ferner wird die Verwendung eines der oben beschriebenen Verfahren zur Herstellung eines der oben beschriebenen medizinischen Clips vorgeschlagen.

Die nachfolgende Beschreibung einer bevorzugten Ausführungsform der Erfindung dient im Zusammenhang mit der Zeichnung der näheren Erläuterung. Es zeigen:
- Figur 1:: eine schematische perspektivische Gesamtansicht eines ersten Ausführungsbeispiels eines medizinischen Clips;
- Figur 2:: eine schematische perspektivische Gesamtansicht eines weiteren Ausführungsbeispiels eines medizinischen Clips;
- Figur 3:: eine schematische Teilansicht eines Werkzeugs zum Vorspannen eines vorspannenden Elements vor dem Anformen von Clipabschnitten mittels eines generativen Herstellungsverfahrens;
- Figur 4:: eine schematische Ansicht ähnlich Figur 3 mit an das vorspannende Element teilweise angeformten Clipabschnitten;
- Figur 5:: eine schematische Ansicht ähnlich Figur 4 mit vollständig angeformten Clipabschnitten;
- Figur 6:: eine schematische Längsschnittansicht eines Ausführungsbeispiels eines Übergangsbereichs zwischen zwei Clipabschnitten;
- Figur 7:: eine schematische Längsschnittansicht eines weiteren Ausführungsbeispiels eines Übergangsbereichs zwischen zwei Clipabschnitten;
- Figur 7a:: eine Querschnittsansicht längs Linie 7a - 7a in Figur 7
- Figur 8:: eine schematische Längsschnittansicht eines weiteren Ausführungsbeispiels eines Übergangsbereichs zwischen zwei Clipabschnitten;
- Figur 8a:: eine Querschnittsansicht längs Linie 8a - 8a in Figur 8;
- Figur 9:: eine schematische Längsschnittansicht eines weiteren Ausführungsbeispiels eines Übergangsbereichs zwischen zwei Clipabschnitten;
- Figur 9a:: eine Querschnittsansicht längs Linie 9a - 9a in Figur 9;
- Figur 10:: eine schematische Längsschnittansicht eines weiteren Ausführungsbeispiels eines Übergangsbereichs zwischen zwei Clipabschnitten;
- Figur 10a:: eine Querschnittsansicht längs Linie 10a - 10a in Figur 10;
- Figur 11:: eine schematische Längsschnittansicht eines weiteren Ausführungsbeispiels eines Übergangsbereichs zwischen zwei Clipabschnitten;
- Figur 11a:: eine Querschnittsansicht längs Linie 11a - 11a in Figur 11;
- Figur 12:: eine schematische perspektivische Gesamtansicht eines weiteren Ausführungsbeispiels eines medizinischen Clips;
- Figur 13:: eine schematische Schnittansicht längs Linie 13 - 13 in Figur 12;
- Figur 14:: eine schematische perspektivische Teilansicht eines weiteren Ausführungsbeispiels eines medizinischen Clips mit einem Doppelstegschluss;
- Figur 15:: eine weitere schematische perspektivische Teilansicht des in Figur 14 dargestellten Ausführungsbeispiels eines medizinischen Clips;
- Figur 16:: eine schematische perspektivische Teilansicht eines weiteren Ausführungsbeispiels eines medizinischen Clips mit einem Doppelstegschluss; und
- Figur 17:: eine weitere schematische perspektivische Teilansicht des in Figur 16 dargestellten Ausführungsbeispiels eines medizinischen Clips.

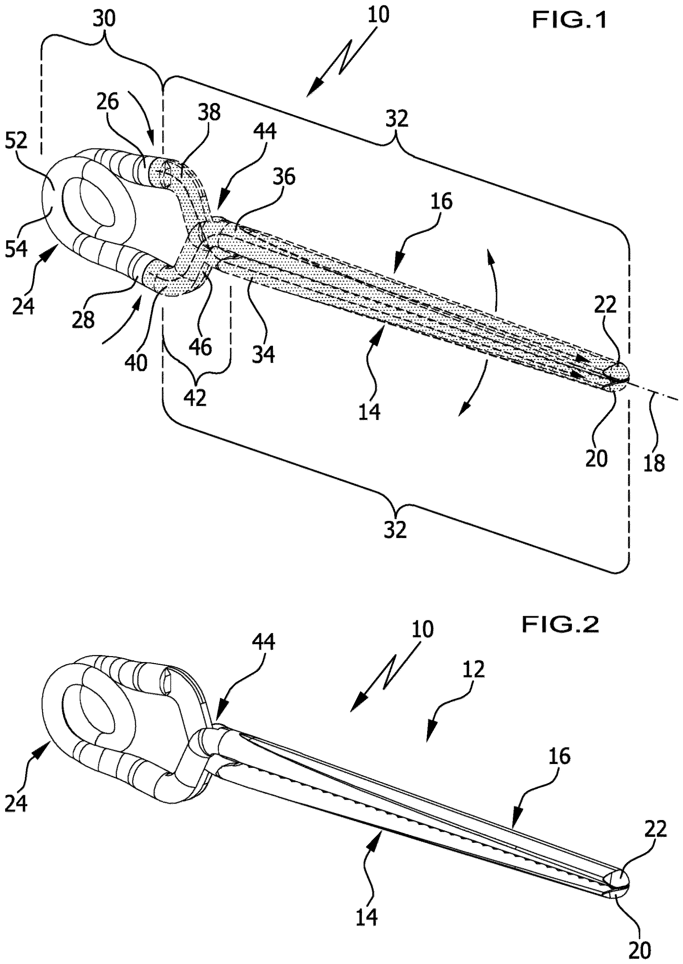

Ein erstes Ausführungsbeispiel eines medizinischen Clips 10 ist schematisch in Figur 1 dargestellt, und zwar in Form eines Aneurysmenclips 12.

Der Clip 10 umfasst einen ersten Klemmarm 14 und einen zweiten Klemmarm 16. Diese sind bei dem in Figur 1 dargestellten Ausführungsbeispiel langgestreckt gerade ausgebildet und definieren in der in Figur 1 dargestellten Grundstellung eine Längsachse 18 des Clips 10.

Die Klemmarme 14 und 16 erstrecken sich distalseitig bis zu freien Enden 20 und 22.

Proximalseitig sind die Klemmarme 14 und 16 mit einem vorspannenden Element 24 verbunden, und zwar der erste Klemmarm 14 mit einem ersten Ende 26 des vorspannenden Elements 24 und der zweite Klemmarm 16 mit einem zweiten Ende 28 des vorspannenden Elements 24.

In der Grundstellung sind die Klemmarme 14 und 16 des Clip 10 einander maximal angenähert. Bei dem in Figur 1 dargestellten Ausführungsbeispiel liegen die Klemmarme 14 und 16 in der Grundstellung aneinander an.

Die Klemmarme 14 und 16 können ausgehend von der Grundstellung in eine Öffnungsstellung voneinander weg bewegt werden. Dies wird erreicht, indem die Enden 26 und 28 aufeinander zu bewegt werden. Dabei wird das vorspannende Element 24 vorgespannt beziehungsweise weiter vorgespannt und die Klemmarme 14 und 16 entgegen der Wirkung des vorspannenden Elements 24 von der Grundstellung in eine in der Figur nicht dargestellte Öffnungsstellung voneinander weg bewegt.

Das beispielhaft in Figur 1 dargestellte Ausführungsbeispiel des Clips 10 umfasst einen ersten Clipabschnitt 30 und zwei zweite Clipabschnitte 32.

Der erste Clipabschnitt 30 ist bei dem dargestellten Ausführungsbeispiel durch einen Kaltumformprozess ausgebildet. Die zwei zweiten Clipabschnitte 32 sind durch ein generatives Herstellungsverfahren direkt an den ersten Clipabschnitt angeformt. Der Clip 10 ist somit teilweise durch ein generatives Herstellungsverfahren ausgebildet.

Der erste Clipabschnitt 30 bildet das vorspannende Element 24. Die zweiten Clipabschnitte 32 sind an die Enden 26 und 28 des vorspannenden Elements 24 direkt angeformt.

Der erste Klemmarm 14 weist ein dem vorspannenden Element 24 zugewandtes Ende 34 auf. Ferner weist der zweite Klemmarm 16 ein dem vorspannenden Element 24 zugewandtes Ende 36 auf. Das Ende 34 ist über einen ersten Verbindungsabschnitt 38 mit dem ersten Ende 26 des vorspannenden Elements 24 verbunden. Das Ende 36 ist über einen zweiten Verbindungsabschnitt 40 mit dem zweiten Ende 28 verbunden.

Bei dem in Figur 1 dargestellten Ausführungsbeispiel sind sowohl die Klemmarme 14 und 16 als auch die beiden Verbindungsabschnitte 38 und 40 durch ein generatives Herstellungsverfahren ausgebildet.

Die Verbindungsabschnitte 38 und 40 kreuzen sich in einem Schlussbereich 42 des Clips 10. Der Schlussbereich 42 ist in Form eines Durchsteckschlusses 44 ausgebildet. Der erste Verbindungsabschnitt 38 bildet dabei ein männliches Teil, welches den zweiten Verbindungsabschnitt 40, der ein weibliches Teil mit einer Schlussbereichsdurchbrechung 46 bildet, durchsetzt.

Der erste Klemmarm 14 weist eine erste Klemmfläche 48 auf. Der zweite Klemmarm 16 weist eine zweite Klemmfläche 50 auf. In der in Figur 1 schematisch dargestellten Grundstellung des Clips 10 liegen die beiden aufeinander zu weisenden Klemmflächen 48 und 50 aneinander an.

Bei dem in Figur 1 dargestellten Ausführungsbeispiel sind in der Grundstellung der erste Klemmarm 14 und der zweite Klemmarm 16 gegeneinander vorgespannt.

Das vorspannende Element 24 ist in Form einer Schraubenfeder 52 ausgebildet, die mehr als eine vollständige Windung aufweist, nämlich etwa 1,5 Windungen.

Die Schraubenfeder 52 ist aus einem Draht 54 durch Kaltumformen ausgebildet.

Der Clip 10 ist aus einem körperverträglichen Werkstoff ausgebildet. Bei dem in Figur 1 dargestellten Ausführungsbeispiel ist der körperverträgliche Werkstoff ein körperverträgliches Metall, nämlich Titan. Sowohl der erste Clipabschnitt 30, der durch Kaltumformen ausgebildet ist, als auch die zweiten Clipabschnitte 32, sind aus Titan ausgebildet.

In Figur 2 ist schematisch ein zweites Ausführungsbeispiel eines insgesamt mit dem Bezugszeichen 10 bezeichneten medizinischen Clips dargestellt. Dieser ist vollständig durch ein generatives Herstellungsverfahren ausgebildet.

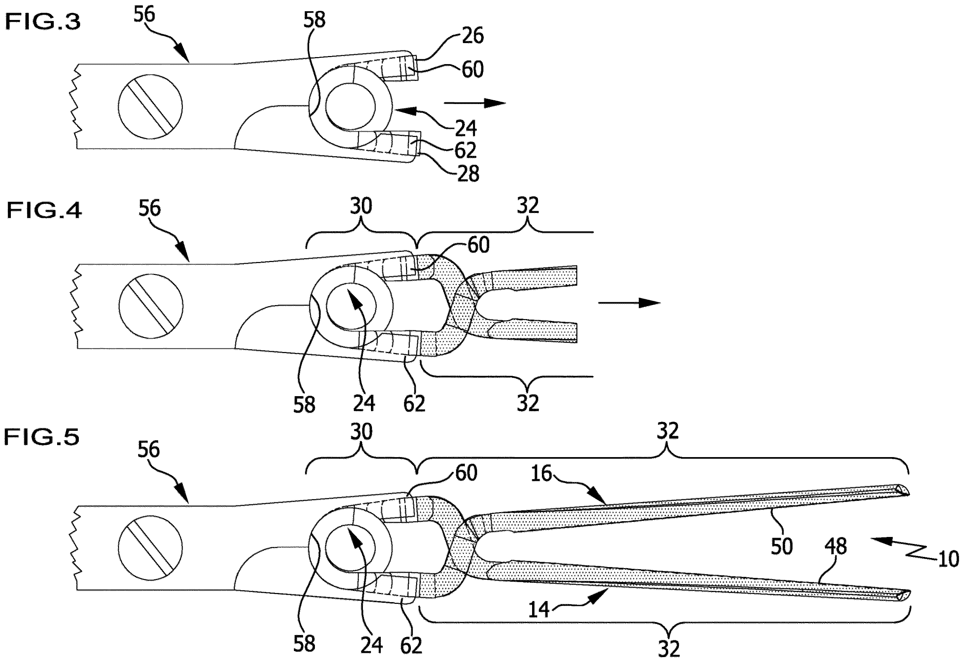

Die Herstellung des Clips 10 wird nachfolgend in Verbindung mit den Figuren 3 bis 5 näher erläutert.

Zum Anformen der zweiten Clipabschnitte 32 an den ersten Clipabschnitt 30 wird der erste Clipabschnitt 30, in Figur 3 schematisch dargestellt in Form des vorspannenden Elements 24, in einem Haltewerkzeug 56 aufgenommen. Mit diesem werden die Enden 26 und 28 etwas aufeinander zu bewegt, sodass das vorspannende Element 24 in einer Haltewerkzeugaufnahme 58 des Haltewerkzeugs 56 unter Vorspannung gehalten ist. Die Enden 26 und 28 weisen in dieser in Figur 3 schematisch dargestellten Anformstellung in distaler Richtung.

Nun können, wie in Figur 4 schematisch dargestellt, mit einem generativen Herstellungsverfahren die zweiten Clipabschnitte 32 sukzessive direkt an den ersten Clipabschnitt 30 angeformt werden.

Als generative Herstellungsverfahren werden zur Ausbildung des Clips 10 wahlweise Laserauftragsschweißen einerseits und ein thermisches Spritverfahren, nämlich ein Metall-Pulver-Auftragsverfahren (MPA), eingesetzt.

In den Figuren 4 und 5 ist gut erkennbar, dass der Clip 10 unter Vorspannung gehalten ist. Die Klemmflächen 48 und 50 sind beim Anformen der Clipabschnitte 32 in einem Abstand voneinander gehalten, und zwar durch das Haltewerkzeug 56, welches das vorspannende Element 24 unter Vorspannung hält.

Ist der Clip 10 wie schematisch in Figur 5 dargestellt, fertig ausgebildet, kann das vorspannende Element 24 aus der Haltewerkzeugaufnahme 58 entnommen werden. Hierfür wird das Haltewerkzeug 56, beziehungsweise dessen zwei Werkzeugenden 60 und 62, die gemeinsam zwischen sich die Haltewerkzeugaufnahme 58 definieren, etwas voneinander weg bewegt, sodass die Klemmarme 14 und 16 aufeinander zu bewegt werden können, bis die Klemmflächen 48 und 50 aneinander anliegen, wie dies schematisch in Figur 1 dargestellt ist.

Zwischen den Clipabschnitten 30 und 32 ist ein Übergangsbereich 64 ausgebildet. Dieser wird definiert durch einen Übergang des ersten Clipabschnitts 30 zum angeformten zweiten Clipabschnitt 32.

Der Übergangsbereich 64 definiert eine Übergangsbereichslängsachse 66. Er erstreckt sich zwischen einem ersten Übergangsbereichsende 68 und einem zweiten Übergangsbereichsende 70. Zwischen dem ersten Übergangsbereichsende 68 und dem zweiten Übergangsbereichsende 70 ist ein Querschnitt quer zur Übergangsbereichslängsachse teilweise durch den ersten Clipabschnitt 30 und teilweise durch den zweiten Clipabschnitt 32 definiert.

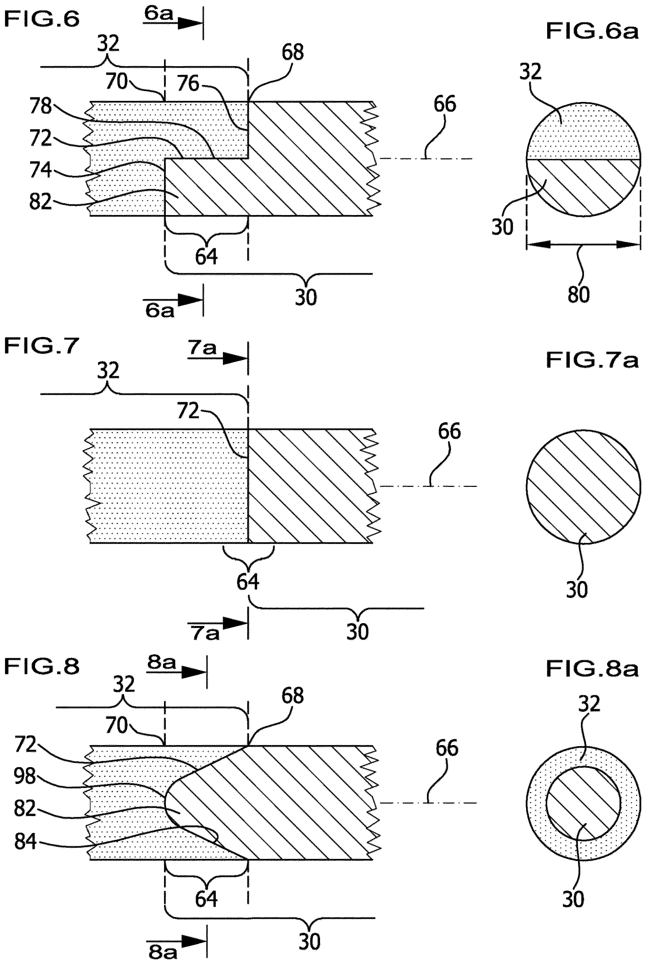

Der Clip 10, wie er beispielhaft in Figur 1 dargestellt ist, weist zwischen den Clipabschnitten 30 und 32 jeweils einen Übergangsbereich 64 auf, welcher in Form eines der in den Figuren 6 bis 11 schematisch dargestellten Übergangsbereiche 64 ausgebildet ist.

Figur 6 zeigt schematisch ein Ausführungsbeispiel des Übergangsbereichs zwischen zwei Clipabschnitten 30 und 32 bei denen im Übergangsbereich 64 zwischen dem ersten Übergangsbereichsende 68 und dem zweiten Übergangsbereichsende 70 ein Querschnitt quer zur Übergangsbereichslängsachse 66 jeweils etwa zur Hälfte durch den ersten Clipabschnitt 30 und den zweiten Clipabschnitt 32 definiert ist.

Figur 6a zeigt schematisch einen Querschnitt zwischen den beiden Übergangsbereichsenden 68 und 70.

Das erste Übergangsbereichsende 68 ist so definiert, dass an diesem ein Querschnitt quer zur Übergangsbereichslängsachse 66 vollständig durch den ersten Clipabschnitt 30 definiert ist. Analog ist das zweite Übergangsbereichsende 70 derart definiert, dass an diesem ein Querschnitt quer zur Übergangsbereichslängsachse 66 vollständig durch den zweiten Clipabschnitt 32 definiert ist.

Im Übergangsbereich 64 ist eine Kontaktfläche 72 zwischen den Clipabschnitten 30 und 32 definiert. Bei dem in Figur 6 dargestellten Ausführungsbeispiel umfasst die Kontaktfläche 72 drei Bereiche, nämlich zwei sich quer zur Übergangsbereichslängsachse 66 erstreckende Querbereiche 74 und 76 sowie einen sich parallel zur Übergangsbereichslängsachse 66 erstreckenden Längsbereich 78.

Eine Größe der Kontaktfläche 72 bei dem in Figur 6 dargestellten Ausführungsbeispiel ist größer als eine Querschnittsfläche des Clips 10 im Übergangsbereich 64 quer zur Übergangsbereichslängsachse 66. Bei dem in Figur 6 dargestellten Ausführungsbeispiel ist die Kontaktfläche 72 gegenüber der Querschnittsfläche des Clips 10 im Übergangsbereich 64 um den Längsbereich 78 vergrößert. Dessen Fläche bestimmt sich als Produkt aus dem Abstand zwischen dem Übergangsbereichsenden 68 und 70 und dem Durchmesser 80 des Clips 10 im Übergangsbereich 64.

Figur 7 zeigt ein weiteres Ausführungsbeispiel eines Übergangsbereichs 64. Hier stoßen im Übergangsbereich 64 die Clipabschnitte 30 und 32 flächig aneinander an. Der Übergangsbereich 64 ist hier beliebig schmal und wird insbesondere vorgegeben durch eine Rauheit von der Kontaktfläche 72 an aufeinander zu weisenden Enden der Clipabschnitte 30 und 32. Die Kontaktfläche 72 entspricht bei diesem Ausführungsbeispiel der Querschnittsfläche des Clips 10 im Übergangsbereich 64 quer zur Übergangsbereichslängsachse 66.

Bei dem in Figur 8 dargestellten Ausführungsbeispiel des Übergangsbereichs 64 ist am Clipabschnitt 30 ein in Richtung auf den Clipabschnitt 32 hin weisender Verbindungsvorsprung 82 ausgebildet. Am Clipabschnitt 32 ist eine Verbindungsvorsprungsaufnahme 84 ausgebildet. Diese nimmt den Verbindungsvorsprung 82 kraft- und/oder form- und/oder stoffschlüssig auf.

Der Verbindungsvorsprung 82 ist bei diesem Ausführungsbeispiel kegelförmig mit abgerundetem Ende ausgebildet.

Die Kontaktfläche 72 ist bei diesem Ausführungsbeispiel größer als die Querschnittsfläche des Clips 10 im Übergangsbereich 64 quer zur Übergangsbereichslängsachse 66.

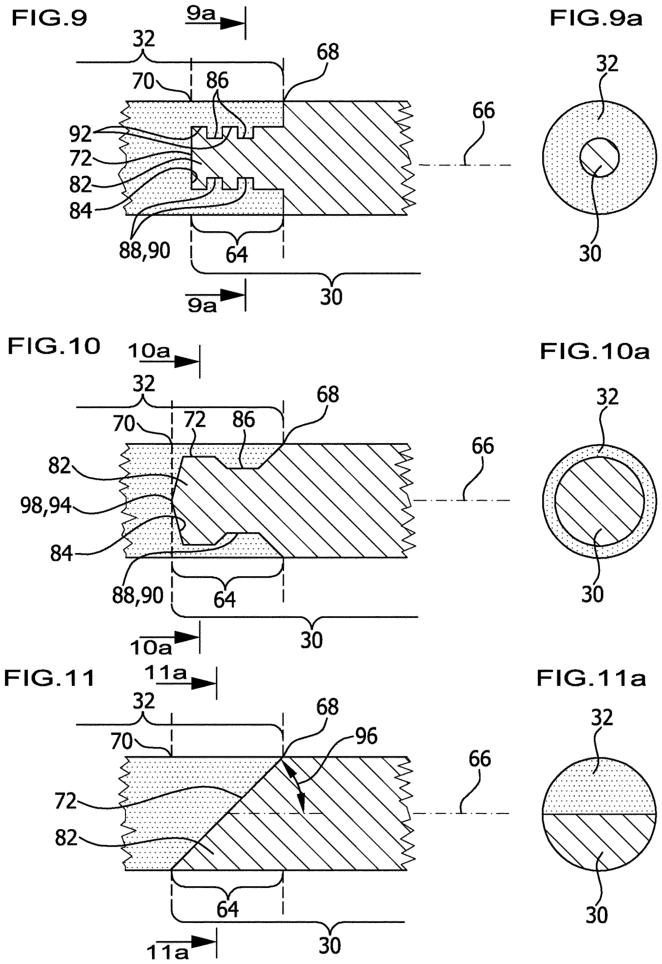

Ein weiteres Ausführungsbeispiel eines Übergangsbereichs 64 ist schematisch in Figur 9 dargestellt. Hier steht vom Clipabschnitt 30 in Richtung auf den Clipabschnitt 32 ein im Wesentlichen zylindrischer Verbindungsvorsprung 82 ab. An diesem sind zwei Hinterschneidungen 86 ausgebildet, welche in einer Richtung quer, nämlich senkrecht, bezogen auf die Übergangsbereichslängsachse 66 geöffnet sind.

Die Hinterschneidungen 68 sind in Form von Nuten 88 ausgebildet, und zwar in Form von Ringnuten 90.

Der Verbindungsvorsprung 82 ist in einer korrespondierend geformten Verbindungsvorsprungsaufnahme 84 aufgenommen. Diese weist korrespondierende Hinterschneidungen auf, und zwar in Form von in Richtung auf die Übergangsbereichslängsachse 66 hin geöffnete Ringnuten 92.

Figur 9a zeigt beispielhaft einen Querschnitt des Übergangsbereichs 64 des Ausführungsbeispiels, das in Figur 9 dargestellt ist.

Ein weiteres Ausführungsbeispiel eines Übergangsbereichs 64 ist schematisch in Figur 10 dargestellt. Auch hier steht vom Clipabschnitt 30 in Richtung auf den Clipabschnitt 32 ein Verbindungsvorsprung 92 ab, welcher kraft- und/oder form- und/oder stoffschlüssig in einer korrespondierend geformten Verbindungsvorsprungsaufnahme 84 aufgenommen ist.

Ausgehend vom ersten Übergangsbereichsende 68 verjüngt sich der Clipabschnitt 30 im Querschnitt kontinuierlich, bleibt dann auf einem kurzen, sich parallel zur Übergangsbereichslängsachs 66 erstreckenden Abschnitt konstant und erweitert sich dann wieder etwas in distaler Richtung kontinuierlich, jedoch nicht in einem Umfang, wie dies durch das erste Übergangsbereichsende 68 definiert wird. Auf einem kurzen Abschnitt bleibt der Querschnitt des Verbindungsvorsprungs 82 in distaler Richtung konstant. Daran anschließend verjüngt er sich kontinuierlich bis zu einem Vorsprungsende 98, welches in Form einer in distaler Richtung weisende Spitze 94 geformt ist.

Wie bereits angemerkt ist die Verbindungsvorsprungsaufnahme 84 in ihrer Form korrespondierend zum Verbindungsvorsprung 82 ausgebildet.

Der Verbindungsvorsprung 82 des in Figur 10 dargestellten Ausführungsbeispiels weist somit ebenfalls eine Hinterschneidung 86 auf, die einer Richtung von der Übergangsbereichslängsachse 66 weg weisend geöffnet ist. Sie ist in Form einer Nut 88, und zwar in Form einer Ringnut 90, ausgebildet.

Figur 11 zeigt schematisch ein weiteres Ausführungsbeispiel eines Übergangsbereichs 64.

Hier ist die Kontaktfläche 72 gegenüber der Übergangsbereichslängsachse 66 um einen Neigungswinkel 96 geneigt, welcher in einem Bereich von etwa 35° bis etwa 55° liegt. Bei dem in Figur 11 dargestellten Ausführungsbeispiel beträgt der Neigungswinkel 96 45°. Die Kontaktfläche 72 ist in diesem Fall eben ausgebildet.

Bei dem in Figur 11 dargestellten Ausführungsbeispiel kann der im Querschnitt abnehmende Clipabschnitt 30 auch als Verbindungsvorsprung 82 bezeichnet werden. Dieser ist, ebenso wie der vom Querbereich 76 abstehende Verbindungsvorsprung 82 bei dem in Figur 6 dargestellten Ausführungsbeispiel des Übergangsbereichs 84 nicht rotationssymmetrisch bezüglich der Übergangsbereichslängsachse 66 ausgebildet.

Dagegen sind die Verbindungsvorsprünge 82 bei den Ausführungsbeispielen der Figuren 8, 9 und 10, ebenso wie die korrespondierenden Verbindungsvorsprungsaufnahmen 84, rotationssymmetrisch bezüglich der Übergangsbereichslängsachse 66 ausgebildet.

Bei dem Ausführungsbeispiel der Figur 7 verläuft die Kontaktfläche 72 vollständig senkrecht zur Übergangsbereichslängsachse 66. Das Ausführungsbeispiel der Figur 11 zeigt eine Kontaktfläche 72, die vollständig quer zur Übergangsbereichslängsachse 66 verläuft, jedoch unter dem Neigungswinkel 96.

Die Kontaktfläche 72 verläuft teilweise parallel und teilweise quer zur Übergangsbereichslängsachse 66 bei den Ausführungsbeispielen der Figuren 6, 8, 9, 10 und 11.

Durch die besondere Ausgestaltung der Übergangsbereiche 64, insbesondere bei den Ausführungsbeispielen der Figuren 6, 8, 9, 10 und 11, ist es möglich, die Kontaktfläche 72 beispielsweise mindestens 50% größer auszubilden als eine Querschnittsfläche des Clips 10 im Übergangsbereich 64 quer zur Übergangsbereichslängsachse 66. Je nach Form und Größe sowie Anzahl der Hinterschneidungen 86 kann die Kontaktfläche sogar mehr als 100% größer sein als die angegebene Querschnittsfläche.

Bei den in den Figuren 6, 8, 9, 10 und 11 dargestellten Ausführungsbeispielen der Übergangsbereiche 64 erstreckt sich dieser über eine Länge, welche mindestens etwa 50% eines Durchmessers des Übergangsbereichs 64 entspricht.

Bei den Ausführungsbeispielen der Figuren 10 und 11 ist die Länge sogar mindestens etwa 100% größer als der Durchmesser 80 des Übergangsbereichs 64.

Bei einem nicht explizit dargestellten Ausführungsbeispiel ist das vorspannende Element durch ein generatives Herstellungsverfahren ausgebildet. An dieses können dann zweite Clipabschnitte 32 ebenfalls durch ein generatives Herstellungsverfahren direkt angeformt werden, wie dies in Verbindung mit den Figuren 3 bis 5 näher erläutert wurde. Auf diese Weise kann ein Clip 10 ausgebildet werden, welcher vollständig durch ein generatives Herstellungsverfahren ausgebildet ist.

Bei einem nicht in den Figuren dargestellten Ausführungsbeispiel ist das vorspannende Element 24 mit den Verbindungsabschnitten 38 und 40 einstückig ausgebildet, und zwar durch ein Kaltumformverfahren.

Bei einem weiteren, in den Figuren nicht dargestellten Ausführungsbeispiel bildet das vorspannende Element 24 mit den Verbindungsabschnitten 38 und 40, die einstückig durch ein generatives Herstellungsverfahren ausgebildet sind, einen ersten Clipabschnitt. An diesen sind dann die Klemmarme 14 und 16, die zweite Clipabschnitte 32 bilden, durch ein generatives Herstellungsverfahren direkt angeformt.

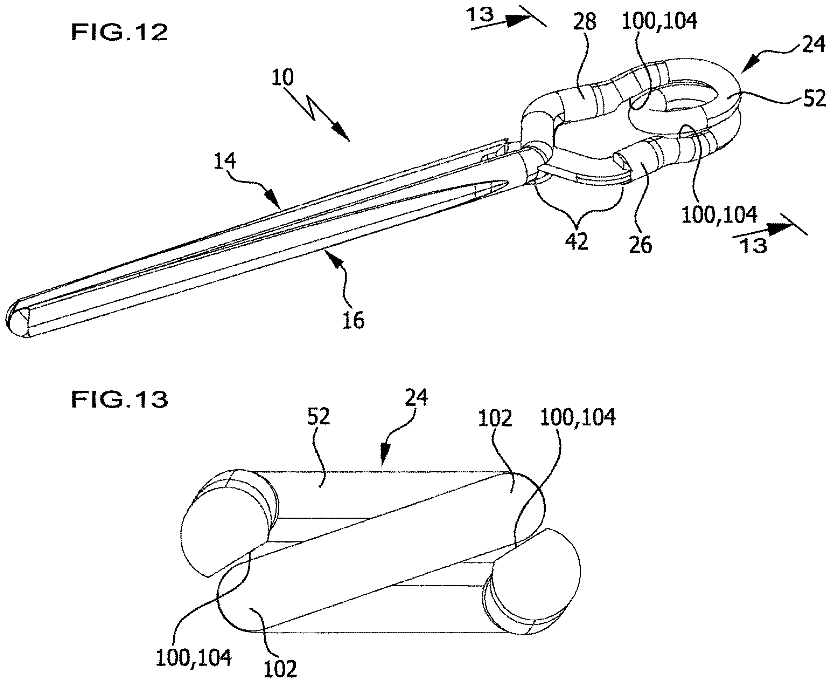

In den Figuren 12 und 13 ist schematisch ein weiteres Ausführungsbeispiel eines medizinischen Clips 10 dargestellt. Dieses Ausführungsbeispiel unterscheidet sich von dem in Figur 1 dargestellten Ausführungsbeispiel insbesondere dadurch, dass am vorspannenden Element 24, welches in Form der Schraubenfeder 52 ausgebildet ist, zwei Windungsaufnahmen 100 ausgebildet sind. Die Windungsaufnahmen 100 sind am vorspannenden Element 24 im Bereich des ersten Endes 26 und des zweiten Endes 28 ausgebildet. Sie sind in Richtung auf die benachbarte Windung 102 hin weisend geöffnet ausgebildet.

Bei dem in den Figuren 12 und 13 dargestellten Ausführungsbeispiel ist die Windungsaufnahme 100 in Form einer Abflachung 104 ausgebildet.

Bei einem nicht dargestellten Ausführungsbeispiel eines Clips 10 ist die Windungsaufnahme 100 in Form einer flachen Ausnehmung am vorspannenden Element 24 ausgebildet. Die flache Ausnehmung ist in Richtung auf die benachbarte Windung 102 hin weisend konkav beziehungsweise schwach konkav gekrümmt.

Die Windungsaufnahme 100 ermöglicht es, die Schraubenfeder 52 im Wesentlichen reibungsfrei zu tordieren, indem die Enden 26 und 28 aufeinander zu bewegt werden. Bei einer solchen Torsion zieht sich die Schraubenfeder 52 etwas zusammen. Ohne die Windungsaufnahmen 100 kann es dabei zu einer Berührung in dem Bereich kommen, in dem bei dem in Figur 12 dargestellten Ausführungsbeispiel des Clips 10 die Windungsaufnahmen 100 vorgesehen sind. Genau diesen Kontakt zu vermeiden ist Aufgabe der Windungsaufnahmen 100. Sie ermöglichen es also, eine mögliche Reibung beim Öffnen des Clips 10 möglichst ganz zu vermeiden. Dies gestattet ist insbesondere, den Clip 10 während und nach dessen Herstellung eingehend zu testen, ohne dass es aufgrund von Reibung im Bereich des vorspannenden Elements 24 zu Verformungen des Clips 10 kommt.

Die in Verbindung mit den Figuren 12 und 13 beschriebene Windungsaufnahme 100 beziehungsweise die beschriebenen Windungsaufnahmen können wahlweise bei einem Clip 10 vorgesehen sein, welcher vollständig durch einen Kaltumformprozess ausgebildet ist.

Alternativ können eine oder zwei Windungsaufnahmen 100 auch bei den verschiedenen Ausführungsbeispielen medizinischer Clips, die in Verbindung mit den Figuren 1 bis 11 erläutert wurden, vorgesehen sein.

Insbesondere ist es möglich, eine oder zwei Windungsaufnahmen 100 an einem vorspannenden Element 24 vorzusehen, welches durch ein generatives Herstellungsverfahren ausgebildet ist.

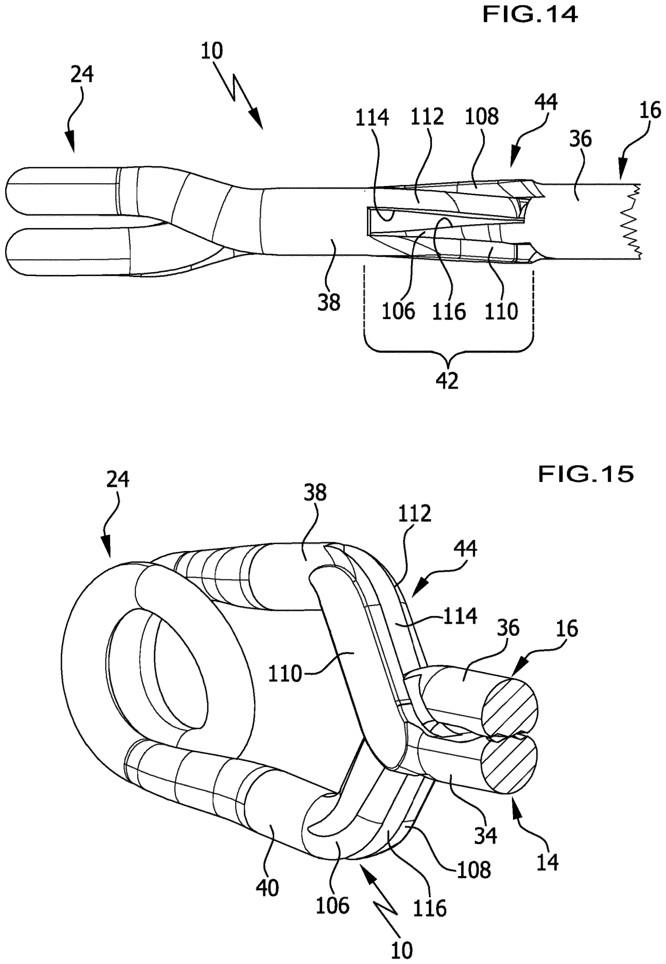

In den Figuren 14 und 15 ist ein weiteres Ausführungsbeispiel eines insgesamt mit dem Bezugszeichen 10 bezeichneten medizinischen Clips teilweise dargestellt. Die Klemmarme 14 und 16 sind bei diesem Ausführungsbeispiel nicht vollständig dargestellt. Sie können insbesondere wie bei den oben beschriebenen Ausführungsbeispielen medizinischer Clips 10 ausgebildet sein.

Bei dem medizinischen Clip 10 gemäß dem Ausführungsbeispiel der Figuren 14 und 15 ist die Ausgestaltung des Schlussbereichs 42 anders als bei den oben beschriebenen Ausführungsbeispielen. Der Schlussbereich 42 ist hier in Form eines sogenannten doppelten Durchsteckschlusses 44 ausgebildet. Dies bedeutet, dass nicht nur der zweite Verbindungsabschnitt 40 mit dem Ende 36 über zwei Stege 106 und 108 verbunden ist, sondern auch der erste Verbindungsabschnitt 38 mit dem Ende 34, und zwar über zwei Stege 110 und 112. Bei den anderen, oben beschriebenen Ausführungsbeispielen der Figuren 1 bis 13 ist nur ein Steg vorgesehen, welcher den Verbindungsabschnitt 38 und das Ende 34 miteinander verbindet. Dieser durchsetzt wie beschrieben die Schlussbereichsdurchbrechnung 46, die von zwei Verbindungsstegen seitlich begrenzt wird, welche den zweiten Verbindungsabschnitt 40 und das Ende 36 miteinander verbinden.

Beim doppelten Durchsteckschluss 44, wie er schematisch in den Figuren 14 und 15 dargestellt ist, durchsetzt der Steg 106 eine zwischen den Stegen 110 und 112 ausgebildete Schlussbereichsdurchbrechung 114. Ferner durchsetzt der Steg 112 eine zwischen den Stegen 106 und 108 definierte Schlussbereichsdurchbrechung 116.

Bei dem Ausführungsbeispiel des medizinischen Clips 10 der Figuren 14 und 15 verlaufen die Stege 106 und 108 parallel zueinander, ebenso die Stege 110 und 112.

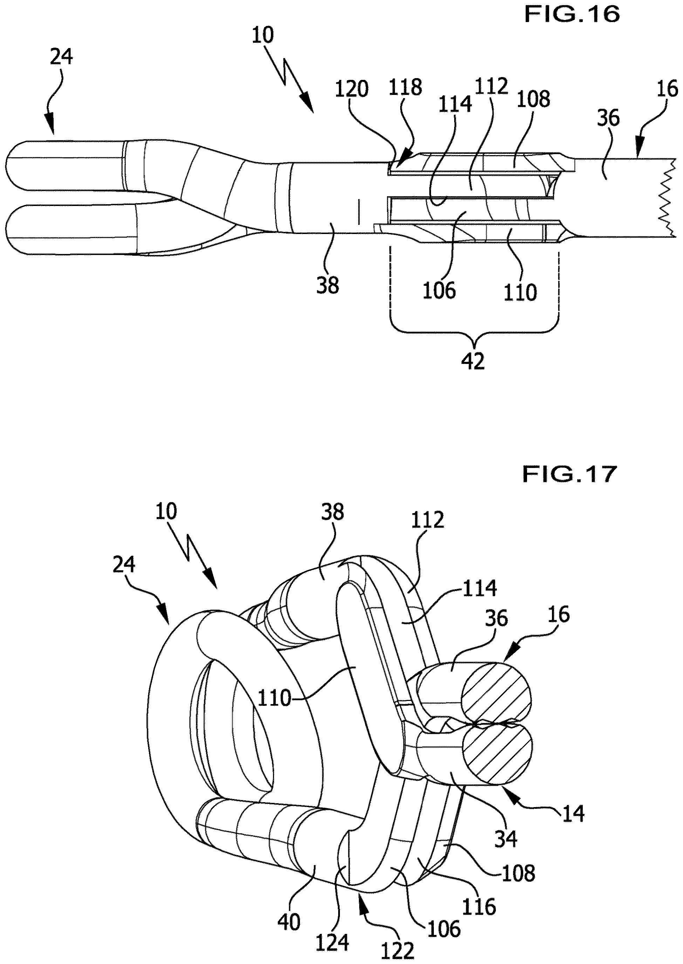

Ein weiteres Ausführungsbeispiel eines medizinischen Clips 10 ist in den Figuren 16 und 17 schematisch in ähnlicher Weise wie das Ausführungsbeispiel der Figuren 14 und 15 dargestellt. Es unterscheidet sich in seinem Aufbau von dem in den Figuren 14 und 15 dargestellten Ausführungsbeispiel lediglich in der Ausgestaltung einer Verbindung der Stege 106 und 108 mit dem Verbindungsabschnitt 40 und der Stege 110 und 112 mit dem Verbindungsabschnitt 38. Wie insbesondere in Figur 16 gut zu erkennen, sind die mit dem Verbindungsabschnitt 38 verbundenen Enden der Stege 110 und 112 etwas seitlich versetzt, so dass im Bereich des Stegs 112 ein Rücksprung 118 ausgebildet ist, welcher eine in distaler Richtung weisende Endfläche 120 am Verbindungsabschnitt 38 definiert. In analoger Weise ist im Übergang zwischen dem Steg 106 und dem zweiten Verbindungsabschnitt 40 ein weiterer Rücksprung 122 ausgebildet, mit einer in distaler Richtung weisenden Endfläche 124.

Das Ausbilden der Rücksprünge 118 und 122 durch ein seitliches Versetzen der Stege 106 und 108 beziehungsweise 110 und 112 im Vergleich zum Ausführungsbeispiel der Figuren 14 und 15 schafft insbesondere Platz für dickere Stege. Für den Fall, dass keine dickeren Stege gewünscht werden, bildet die alternative Anbindung der Stege 106 und 108 an den zweiten Verbindungsabschnitt 40 und die Anbindung der Stege 110 und 112 an den ersten Verbindungsabschnitt 38 die Option, dass die im Querschnitt im Wesentlichen rechteckigen Stege 106 und 112 über die Verbindungsabschnitte 38 und 40, die aus einem im Querschnitt kreisförmigen oder im Wesentlichen kreisförmigen Draht ausgebildet sind, seitlich weniger weit vorstehen als beim Ausführungsbeispiel der Figuren 14 und 15.

Die Rücksprünge 118 und 122 verhindern insbesondere, dass die Verbindungsabschnitte 38 und 40 in dafür vorgesehenen Aufnahmen eines in den Figuren nicht dargestellten Clipanlegeinstruments nicht vollständig aufgenommen werden können. Seitliche Vorsprünge, Auswölbungen oder Wulste in den Bereichen, in denen die Rücksprünge 118 und 122 ausgebildet sind, können im ungünstigsten Fall dazu führen, dass die Verbindungsabschnitte 38 und 40 derartiger medizinischer Clips, wie beispielsweise die Verbindungsabschnitte 38 und 40 des in den Figuren 14 und 15 dargestellten Ausführungsbeispiels des medizinischen Clips 10, in unerwünschter Weise aus am Clipanlegeinstrument vorgesehenen Aufnahmen für die Verbindungsabschnitte herausgedrückt werden. Dies wird durch die besondere Ausgestaltung des medizinischen Clips 10 gemäß den Figuren 16 und 17 verhindert.

Die im Vergleich zum Ausführungsbeispiel der Figuren 14 und 15 schräg verlaufenden Stege 106 und 108 einerseits und 110 und 112 bei dem in den Figuren 16 und 17 dargestellten Ausführungsbeispiel sind wie beschrieben nur an ihren mit den Verbindungsabschnitten 38 und 40 verbundenen Enden schräg bezüglich einer senkrecht beziehungsweise im Wesentlichen senkrecht zu einer vom vorspannenden Element 24 definierten Clipebene verlaufend. Auf der anderen Seite, also dort, wo die anderen Enden der Stege 106 und 108 beziehungsweise 110 und 112 mit den Enden 36 beziehungsweise 34 verbunden sind, bringt ein Versatz keinen Vorteil, da der Clip 10 nur mit den Verbindungsabschnitten 38 und 40 am Clipanlegeinstrument anliegt, nicht jedoch mit den Klemmarmen 14 und 16. Überdies wäre ein Versatz der Stege 106 und 108 beziehungsweise 110 und 112 an den Enden 36 und 34 sogar kontraproduktiv, weil dann die Klemmarme 14 und 16 nicht mehr in einer Ebene liegen würden. Sie müssten dann zusätzlich durch eine Kröpfung oder eine Bajonettform wieder in eine parallele Stellung gebracht werden.

Die Ausführungsbeispiele der Figuren 14 bis 17 sollen lediglich der Erläuterung des doppelten Durchsteckschlusses 44 dienen. Ihre weiteren Komponenten können insbesondere ausgebildet sein, wie dies oben in Verbindung mit den Figuren 1 bis 13 im Detail erläutert ist.

Die oben beschriebenen Clips 10, insbesondere die in Verbindung mit den Figuren 1 bis 17 beschriebenen Clips 10, die teilweise oder vollständig durch ein generatives Herstellungsverfahren ausgebildet sind, ermöglichen insbesondere eine kostengünstige Herstellung für eine große Variantenvielfalt von medizinischen Clips 10.

Die in den Figuren dargestellte Form der Klemmarme 14 und 16 ist rein beispielhaft. Statt geradlinig verlaufender Klemmarme 14 und 16 können hier grundsätzlich beliebig geformte Klemmarme 14 und 16 vorgesehen werden.

Die Herstellung insbesondere der zweiten Clipabschnitte 32 eröffnet die Möglichkeit zur patientenspezifischen Fertigung medizinischer Clips. Ferner kann durch eine Kombination von vorspannenden Elementen 24, die durch ein Kaltumformverfahren ausgebildet sind, eine hohe Festigkeit erreicht werden. Insbesondere die Ausbildung des Schlussbereichs 42 durch ein generatives Herstellungsverfahren ermöglicht eine Reduzierung der Prozessschritte zur Herstellung des Clips 10.

Durch verschiedene vorspannende Elemente 24, insbesondere unterschiedlicher Größe und mit unterschiedlichen Federkonstanten, muss nur eine geringe Anzahl vorspannender Elemente unterschiedlicher Art und Form vorgehalten werden, um letztlich eine beliebige Anzahl unterschiedlicher Varianten medizinischer Clips ausbilden zu können. Insbesondere Form und Größe der Klemmarme 14 und 16 kann bei Bedarf frei vorgegeben und realisiert werden.

Durch Vorspannen des vorspannenden Elements 24 beim Anformen der zweiten Clipabschnitte, wie oben insbesondere in Verbindung mit den Figuren 3 bis 5 erläutert, kann eine Schließkraft des Clips 10 bereits beim Fertigungsprozess in gewünschter Weise eingestellt werden.

### Bezugszeichenliste

- 10: medizinischer Clip
- 12: Aneurysmenclip
- 14: erster Klemmarm
- 16: zweiter Klemmarm
- 18: Längsachse
- 20: freies Ende
- 22: freies Ende
- 24: vorspannendes Element
- 26: erstes Ende
- 28: zweites Ende
- 30: erster Clipabschnitt
- 32: zweiter Clipabschnitt
- 34: Ende
- 36: Ende
- 38: erster Verbindungsabschnitt
- 40: zweiter Verbindungsabschnitt
- 42: Schlussbereich
- 44: Durchsteckschluss
- 46: Schlussbereichsdurchbrechung
- 48: erste Klemmfläche
- 50: zweite Klemmfläche
- 52: Schraubenfeder
- 54: Draht
- 56: Haltewerkzeug
- 58: Haltewerkzeugaufnahme
- 60: Werkzeugende
- 62: Werkzeugende
- 64: Übergangsbereich
- 66: Übergangsbereichslängsachse
- 68: erstes Übergangsbereichsende
- 70: zweites Übergangsbereichsende
- 72: Kontaktfläche
- 74: Querbereich
- 76: Querbereich
- 78: Längsbereich
- 80: Durchmesser
- 82: Verbindungsvorsprung
- 84: Verbindungsvorsprungsaufnahme
- 86: Hinterschneidung
- 88: Nut
- 90: Ringnut
- 92: Ringnut
- 94: Spitze
- 96: Neigungswinkel
- 98: Vorsprungsende
- 100: Windungsaufnahme
- 102: Windung
- 104: Abflachung
- 106: Steg
- 108: Steg
- 110: Steg
- 112: Steg
- 114: Schlussbereichsdurchbrechung
- 116: Schlussbereichsdurchbrechung
- 118: Rücksprung
- 120: Endfläche
- 122: Rücksprung
- 124: Endfläche

## Patentansprüche

1. Medizinischer Clip (10), insbesondere in Form eines Aneurysmenclips (12), welcher einen ersten Klemmarm (14), einen zweiten Klemmarm (16) und ein vorspannendes Element (24) umfasst, wobei der erste Klemmarm (14) mit einem ersten Ende (26) des vorspannenden Elements (24) verbunden ist, wobei der zweite Klemmarm (16) mit einem zweiten Ende (28) des vorspannenden Elements (24) verbunden ist und wobei der erste Klemmarm (14) und der zweite Klemmarm (16) in einer Grundstellung des Clips (10) einander maximal angenähert sind, insbesondere aneinander anliegend, und entgegen der Wirkung des vorspannenden Elements (24) von der Grundstellung in eine Öffnungsstellung voneinander weg bewegbar sind, wobei der medizinische Clip (10) mindestens einen ersten Clipabschnitt (30) und mindestens einen zweiten Clipabschnitt (32) umfasst, **dadurch gekennzeichnet, dass** der medizinische Clip (10) teilweise durch ein generatives Herstellungsverfahren ausgebildet ist und dass der mindestens eine zweite Clipabschnitt (32) durch ein generatives Herstellungsverfahren direkt an den mindestens einen ersten Clipabschnitt (30) angeformt ist.

2. Medizinischer Clip nach Anspruch 1, **dadurch gekennzeichnet, dass** der erste Klemmarm (14) und/oder der zweite Klemmarm (16) durch ein generatives Herstellungsverfahren ausgebildet sind.

3. Medizinischer Clip nach einem der voranstehenden Ansprüche, **dadurch gekennzeichnet, dass** das vorspannende Element (24) durch ein generatives Herstellungsverfahren ausgebildet ist.

4. Medizinischer Clip nach einem der voranstehenden Ansprüche, **dadurch gekennzeichnet, dass** der erste Klemmarm (14) ein dem vorspannenden Element (24) zugewandtes Ende (34) aufweist, dass der zweite Klemmarm (16) ein dem vorspannenden Element (24) zugewandtes Ende (36) aufweist, dass das dem vorspannenden Element (24) zugewandte Ende (34) des ersten Klemmarms (14) über einen ersten Verbindungsabschnitt (38) mit dem ersten Ende (26) des vorspannenden Elements (24) verbunden ist und dass das dem vorspannenden Element (24) zugewandte Ende (36) des zweiten Klemmarms (16) über einen zweiten Verbindungsabschnitt (40) mit dem zweiten Ende (28) des vorspannenden Elements (24) verbunden ist.

5. Medizinischer Clip nach Anspruch 4, **dadurch gekennzeichnet, dass**
a) sich der erste Verbindungsabschnitt (38) und der zweite Verbindungsabschnitt (40) in einem Schlussbereich (42) des Clips (10) kreuzen,
wobei insbesondere der Schlussbereich (42) in Form eines Durchsteckschlusses (44) ausgebildet ist,
und/oder
b) der eine der zwei Verbindungsabschnitte (38, 40) mit einer Schlussbereichsdurchbrechung (46) ausgebildet ist und dass der andere der zwei Verbindungsabschnitte (38, 40) die Schlussbereichsdurchbrechung (46) durchsetzt
und/oder
c) der erste Verbindungsabschnitt (38) und/oder der zweite Verbindungsabschnitt (40) durch spanende Bearbeitung ausgebildet sind
und/oder
d) der erste Verbindungsabschnitt (38) und/oder der zweite Verbindungsabschnitt (40) durch ein generatives Herstellungsverfahren ausgebildet sind.

6. Medizinischer Clip nach einem der voranstehenden Ansprüche, **dadurch gekennzeichnet, dass** der erste Klemmarm (14) eine erste Klemmfläche (48) aufweist, dass der zweite Klemmarm (16) eine zweite Klemmfläche (50) aufweist und dass in der Grundstellung die erste Klemmfläche (48) und die zweite Klemmfläche (50) aneinander anliegen oder im Wesentlichen aneinander anliegen.

7. Medizinischer Clip nach einem der voranstehenden Ansprüche, **dadurch gekennzeichnet, dass**
a) der erste Klemmarm (14) und der zweite Klemmarm (16) in der Grundstellung gegeneinander vorgespannt sind
und/oder
b) das vorspannende Element (24) durch einen Kaltumformprozess ausgebildet ist.

8. Medizinischer Clip nach einem der voranstehenden Ansprüche, **dadurch gekennzeichnet, dass**
a) der Clip (10) aus mindestens einem körperverträglichen Werkstoff, insbesondere nur aus einem einzigen körperverträglichen Werkstoff, ausgebildet ist
und/oder
b) der mindestens eine erste Clipabschnitt (30) durch einen Kaltumformprozess oder durch ein generatives Herstellungsverfahren ausgebildet ist.

9. Medizinischer Clip nach einem der voranstehenden Ansprüche, **dadurch gekennzeichnet, dass** ein Übergang des mindestens einen ersten Clipabschnitts (30) zum mindestens einen zweiten Clipabschnitt (32) einen Übergangsbereich (64) definiert.

10. Medizinischer Clip nach Anspruch 9, **dadurch gekennzeichnet, dass** der Übergangsbereich (64)
a) eine Übergangsbereichslängsachse (66) definiert und sich zwischen einem ersten Übergangsbereichsende (68) und einem zweiten Übergangsbereichsende (70) erstreckt, dass zwischen dem ersten Übergangsbereichsende (68) und dem zweiten Übergangsbereichsende (70) ein Querschnitt quer zur Übergangsbereichslängsachse (66) teilweise durch den mindestens einen ersten Clipabschnitt (30) und teilweise durch den mindestens einen zweiten Clipabschnitt (32) definiert ist,
wobei insbesondere am ersten Übergangsbereichsende (68) ein Querschnitt quer zur Übergangsbereichslängsachse (66) vollständig durch den mindestens einen ersten Clipabschnitt (30) definiert ist und wobei am zweiten Übergangsbereichsende (70) ein Querschnitt quer zur Übergangsbereichslängsachse (66) vollständig durch den mindestens einen zweiten Clipabschnitt (32) definiert ist,
und/oder
b) eine Kontaktfläche (72) zwischen dem mindestens einen ersten Clipabschnitt (30) und dem mindestens einen zweiten Clipabschnitt (32) definiert und dass die Kontaktfläche (72) mindestens einer Querschnittsfläche des Clips (10) im Übergangsbereich (64) quer zur Übergangsbereichslängsachse (66) entspricht,
wobei insbesondere die Kontaktfläche (72) mindestens 50% größer ist als eine Querschnittsfläche des Clips (10) im Übergangsbereich (64) quer zur Übergangsbereichslängsachse (66), insbesondere mindestens 100% größer.

11. Medizinischer Clip nach Anspruch 9 oder 10, **dadurch gekennzeichnet, dass** an einem der Clipabschnitte (30, 32) ein sich in Richtung auf den mindestens einen anderen Clipabschnitt (30, 32) hin weisender Verbindungsvorsprung (82) ausgebildet ist und dass am mindestens einen anderen Clipabschnitt (30, 32) eine Verbindungsvorsprungsaufnahme (84) ausgebildet ist, die den Verbindungsvorsprung (82) kraft- und/oder form- und/oder stoffschlüssig aufnimmt,
wobei insbesondere am Verbindungsvorsprung (82) und/oder an der Verbindungsvorsprungsaufnahme (84) mindestens eine Hinterschneidung (86) ausgebildet ist.

12. Medizinischer Clip (10) nach einem der voranstehenden Ansprüche, **dadurch gekennzeichnet, dass** das vorspannende Element (24) in Form einer Schraubenfeder (52) mit mindestens einer vollständigen Windung ausgebildet ist und dass am vorspannenden Element (24) im Bereich des ersten Endes (26) und/oder des zweiten Endes (28) eine Windungsaufnahme (100) ausgebildet ist und dass die Windungsaufnahme (100) in Richtung auf die benachbarte Windung (102) weisend ausgebildet ist, wobei insbesondere die Windungsaufnahme (100) durch eine Abflachung (104) oder eine flache Ausnehmung am vorspannenden Element (24) ausgebildet ist.

13. Verfahren zum Herstellen eines medizinischen Clips (10), insbesondere in Form eines Aneurysmenclips (12), wobei der Clip (10) mit einem ersten Klemmarm (14), einem zweiten Klemmarm (16) und einem vorspannenden Element (24) ausgebildet wird derart, dass der erste Klemmarm (14) mit einem ersten Ende (26) des vorspannenden Elements (24) verbunden ist, dass der zweite Klemmarm (16) mit einem zweiten Ende (28) des vorspannenden Elements (24) verbunden ist und dass der erste Klemmarm (14) und der zweite Klemmarm (16) in einer Grundstellung des Clips (10) einander maximal angenähert sind, insbesondere aneinander anliegend, und entgegen der Wirkung des vorspannenden Elements (24) von der Grundstellung in eine Öffnungsstellung voneinander weg bewegbar sind, wobei der medizinische Clip (10) mit mindestens einem ersten Clipabschnitt (30) und mindestens einem zweiten Clipabschnitt (32) ausgebildet wird, **dadurch gekennzeichnet, dass** der medizinische Clip (10) teilweise durch ein generatives Herstellungsverfahren ausgebildet wird und dass der mindestens eine zweite Clipabschnitt (32) durch ein generatives Herstellungsverfahren direkt an den mindestens einen ersten Clipabschnitt (30) angeformt wird.

14. Verfahren nach Anspruch 13, **dadurch gekennzeichnet, dass** der mindestens eine erste Clipabschnitt (30)
a) durch einen Kaltumformprozess oder durch ein generatives Herstellungsverfahren ausgebildet wird
und/oder
b) in Form des vorspannenden Elements (24) ausgebildet wird und dass das vorspannende Element (24) beim Anformen des zweiten Clipabschnitts (32) unter Vorspannung gehalten wird, insbesondere durch Tordieren der Schraubenfeder (52).

15. Verwendung eines Verfahrens nach Anspruch 13 oder 14 zur Herstellung eines medizinischen Clips (10) nach einem der Ansprüche 1 bis 12.

## Claims

1. Medical clip (10), in particular in the form of an aneurysm clip (12), which comprises a first clamping arm (14), a second clamping arm (16), and a biasing element (24), wherein the first clamping arm (14) is connected to a first end (26) of the biasing element (24), wherein the second clamping arm (16) is connected to a second end (28) of the biasing element (24), and wherein the first clamping arm (14) and the second clamping arm (16) are maximally proximate to one another, in particular abutting against one another, in a basic position of the clip (10) and are movable away from one another against the action of the biasing element (24) from the basic position into an opening position, wherein the medical clip (10) comprises at least one first clip portion (30) and at least one second clip portion (32), **characterized in that** the medical clip (10) is made partially by a generative manufacturing process, and **in that** the at least one second clip portion (32) is formed directly onto the at least one first clip portion (30) by a generative manufacturing process.

2. Medical clip in accordance with Claim 1, **characterized in that** the first clamping arm (14) and/or the second clamping arm (16) are formed by a generative manufacturing process.

3. Medical clip in accordance with any one of the preceding Claims, **characterized in that** the biasing element (24) is formed by a generative manufacturing process.

4. Medical clip in accordance with any one of the preceding Claims, **characterized in that** the first clamping arm (14) comprises an end (34) pointing toward the biasing element (24), **in that** the second clamping arm (16) comprises an end (36) pointing toward the biasing element (24), **in that** the end (34) of the first clamping arm (14) pointing toward the biasing element (24) is connected to the first end (26) of the biasing element (24) by way of a first connecting portion (38), and **in that** the end (36) of the second clamping arm (16) pointing toward the biasing element (24) is connected to the second end (28) of the biasing element (24) by way of a second connecting portion (40).

5. Medical clip in accordance with Claim 4, **characterized in that**
a) the first connecting portion (38) and the second connecting portion (40) intersect in a connection region (42) of the clip (10), wherein, in particular, the connection region (42) is configured in the form of a box lock (44),
and/or
b) the one of the two connecting portions (38, 40) is configured having a connection region perforation (46) and **in that** the other one of the two connecting portions (38, 40) passes through the connection region perforation (46)
and/or
c) the first connecting portion (38) and/or the second connecting portion (40) are formed by machining
and/or
d) the first connecting portion (38) and/or the second connecting portion (40) are formed by a generative manufacturing process.

6. Medical clip in accordance with any one of the preceding Claims, **characterized in that** the first clamping arm (14) comprises a first clamping face (48), **in that** the second clamping arm (16) comprises a second clamping face (50), and **in that** the first clamping face (48) and the second clamping face (50) abut against one another or substantially abut against one another in the basic position.

7. Medical clip in accordance with any one of the preceding Claims, **characterized in that**
a) the first clamping arm (14) and the second clamping arm (16) are biased against one another in the basic position,
and/or
b) the biasing element (24) is formed by a cold forming process.

8. Medical clip in accordance with any one of the preceding Claims, **characterized in that**
a) the clip (10) is made of at least one biocompatible material, in particular only of one single biocompatible material,
and/or
b) the at least one first clip portion (30) is formed by a cold forming process or by a generative manufacturing process.

9. Medical clip in accordance with any one of the preceding Claims, **characterized in that** a transition of the at least one first clip portion (30) to the at least one second clip portion (32) defines a transition region (64).

10. Medical clip in accordance with Claim 9, **characterized in that** the transition region (64)
a) defines a transition region longitudinal axis (66) and extends between a first transition region end (68) and a second transition region end (70), **in that** between the first transition region end (68) and the second transition region end (70) a cross section transverse to the transition region longitudinal axis (66) is defined partially by the at least one first clip portion (30) and partially by the at least one second clip portion (32),
wherein, in particular, at the first transition region end (68) a cross section transverse to the transition region longitudinal axis (66) is defined completely by the at least one first clip portion (30), and wherein at the second transition region end (70) a cross section transverse to the transition region longitudinal axis (66) is defined completely by the at least one second clip portion (32),
and/or
b) defines a contact face (72) between the at least one first clip portion (30) and the at least one second clip portion (32) and **in that** the contact face (72) corresponds at least to a cross sectional area of the clip (10) in the transition region (64) transverse to the transition region longitudinal axis (66),
wherein, in particular, the contact face (72) is at least 50% larger than a cross sectional area of the clip (10) in the transition region (64) transverse to the transition region longitudinal axis (66), in particular at least 100% larger.

11. Medical clip in accordance with Claim 9 or 10, **characterized in that** formed on one of the clip portions (30, 32) is a connecting projection (82) pointing in the direction toward the at least one other clip portion (30, 32) and **in that** formed on the at least one other clip portion (30, 32) is a connecting projection receptacle (84), which accommodates the connecting projection (82) in a force-locking and/or positive-locking and/or materially bonded manner,
wherein, in particular, at least one undercut (86) is formed on the connecting projection (82) and/or on the connecting projection receptacle (84).

12. Medical clip (10) in accordance with any one of the preceding Claims, **characterized in that** the biasing element (24) is configured in the form of a coil spring (52) with at least one complete winding, and **in that** a winding receptacle (100) is formed on the biasing element (24) in the region of the first end (26) and/or the second end (28), and **in that** the winding receptacle (100) is formed pointing in the direction toward the adjacent winding (102),
wherein, in particular the winding receptacle (100) is formed by a flattened portion (104) or a flat recess on the biasing element (24).

13. Method for producing a medical clip (10), in particular in the form of an aneurysm clip (12), wherein the clip (10) is configured having a first clamping arm (14), a second clamping arm (16), and a biasing element (24), such that the first clamping arm (14) is connected to a first end (26) of the biasing element (24), that the second clamping arm (16) is connected to a second end (28) of the biasing element (24), and that the first clamping arm (14) and the second clamping arm (16) are maximally proximate to one another, in particular abutting against one another, in a basic position of the clip (10), and are movable away from one another against the action of the biasing element (24) from the basic position into an opening position, wherein the medical clip (10) is configured having at least one first clip portion (30) and at least one second clip portion (32), **characterized in that** the medical clip (10) is formed partially by a generative manufacturing process, and **in that** the at least one second clip portion (32) is formed directly onto the at least one first clip portion (30) by a generative manufacturing process.

14. Method in accordance with Claim 13, **characterized in that** the at least one first clip portion (30)
a) is formed by a cold forming process or by a generative manufacturing process
and/or
b) is configured in the form of the biasing element (24) and **in that** the biasing element (24) is held under pretension, in particular by twisting the coil spring (52), when forming on the second clip portion (32).

15. Use of a method in accordance with Claim 13 or 14 for producing a medical clip (10) in accordance with any one of Claims 1 to 12.

## Revendications

1. Clip médical (10), en particulier sous la forme d'un clip pour anévrisme (12), lequel comprend un premier bras de serrage (14), un deuxième bras de serrage (16) et un élément de précontrainte (24), dans lequel le premier bras de serrage (14) est relié à une première extrémité (26) de l'élément de précontrainte (24), dans lequel le deuxième bras de serrage (16) est relié à une deuxième extrémité (28) de l'élément de précontrainte (24) et dans lequel le premier bras de serrage (14) et le deuxième bras de serrage (16) dans une position de base du clip (10) sont rapprochés au maximum l'un de l'autre, en particulier de manière à s'appliquer l'un sur l'autre, et à l'encontre de l'action de l'élément de précontrainte (24) peuvent s'éloigner l'un de l'autre à partir de la position de base dans une position ouverte, le clip médical (10) comprenant au moins une première partie de clip (30) et au moins une deuxième partie de clip (32), **caractérisé en ce que** le clip médical (10) est réalisé en partie par un procédé de fabrication additive et que la au moins une deuxième partie de clip (32) est formée directement sur la au moins une première partie de clip (30) par un procédé de fabrication additive.

2. Clip médical selon la revendication 1, **caractérisé en ce que** le premier bras de serrage (14) et/ou le deuxième bras de serrage (16) sont réalisés par un procédé de fabrication additive.

3. Clip médical selon l'une quelconque des revendications précédentes, **caractérisé en ce que** l'élément de précontrainte (24) est réalisé par un procédé de fabrication additive.

4. Clip médical selon l'une quelconque des revendications précédentes, **caractérisé en ce que** le premier bras de serrage (14) présente une extrémité (34) tournée vers l'élément de précontrainte (24), **en ce que** le deuxième bras de serrage (16) présente une extrémité (36) tournée vers l'élément de précontrainte (24), **en ce que** l'extrémité (34) du premier bras de serrage (14) tournée vers l'élément de précontrainte (24) est reliée à la première extrémité (26) de l'élément de précontrainte (24) par l'intermédiaire d'une première partie de liaison (38) et **en ce que** l'extrémité (36) du deuxième bras de serrage (16) tournée vers l'élément de précontrainte (24) est reliée à la deuxième extrémité (28) de l'élément de précontrainte (24) par l'intermédiaire d'une deuxième partie de liaison (40).

5. Clip médical selon la revendication 4, **caractérisé en ce que**
a) la première partie de liaison (38) et la deuxième partie de liaison (40) se croisent dans une zone de fermeture (42) du clip (10), dans lequel en particulier la zone de fermeture (42) est réalisée sous la forme d'une fermeture traversante (44),
et/ou
b) l'une des deux parties de liaison (38, 40) est réalisée avec une ouverture de zone de fermeture (46) et **en ce que** l'autre des deux parties de liaison (38, 40) traverse l'ouverture de zone de fermeture (46)
et/ou
c) la première partie de liaison (38) et/ou la deuxième partie de liaison (40) sont réalisées par usinage par enlèvement de copeaux
et/ou
d) la première partie de liaison (38) et/ou la deuxième partie de liaison (40) sont réalisées par un procédé de fabrication additive.

6. Clip médical selon l'une quelconque des revendications précédentes, **caractérisé en ce que** le premier bras de serrage (14) présente une première surface de serrage (48), **en ce que** le deuxième bras de serrage (16) présente une deuxième surface de serrage (50) et **en ce que** dans la position de base la première surface de serrage (48) et la deuxième surface de serrage (50) s'appliquent l'une sur l'autre ou s'appliquent sensiblement l'une sur l'autre.

7. Clip médical selon l'une quelconque des revendications précédentes, **caractérisé en ce que**
a) le premier bras de serrage (14) et le deuxième bras de serrage (16) sont précontraints l'un par rapport à l'autre dans la position de base
et/ou
b) l'élément de précontrainte (24) est réalisé par un processus de formage à froid.

8. Clip médical selon l'une quelconque des revendications précédentes, **caractérisé en ce que**
a) le clip (10) est réalisé à partir d'au moins un matériau toléré par le corps, en particulier uniquement à partir d'un seul matériau toléré par le corps,
et/ou
b) la au moins une première partie de clip (30) est réalisée par un processus de formage à froid ou par un procédé de fabrication additive.

9. Clip médical selon l'une quelconque des revendications précédentes, **caractérisé en ce qu'**un passage de la au moins une première partie de clip (30) vers la au moins une deuxième partie de clip (32) définit une zone de passage (64).

10. Clip médical selon la revendication 9, **caractérisé en ce que** la zone de passage (64)
a) définit un axe longitudinal de zone de passage (66) et s'étend entre une première extrémité de zone de passage (68) et une deuxième extrémité de zone de passage (70), **en ce qu'**entre la première extrémité de zone de passage (68) et la deuxième extrémité de zone de passage (70) une section transversale transversale par rapport à l'axe longitudinal de zone de passage (66) est définie en partie par la au moins une première partie de clip (30) et en partie par la au moins une deuxième partie de clip (32),
dans lequel en particulier sur la première extrémité de zone de passage (68) une section transversale transversale par rapport à l'axe longitudinal de zone de passage (66) est entièrement définie par la au moins une première partie de clip (30) et dans lequel sur la deuxième extrémité de zone de passage (70) une section transversale transversale par rapport à l'axe longitudinal de zone de passage (66) est entièrement définie par la au moins une deuxième partie de clip (32),
et/ou
b) définit une surface de contact (72) entre la au moins une première partie de clip (30) et la au moins une deuxième partie de clip (32) et **en ce que** la surface de contact (72) correspond au moins à une surface de section transversale du clip (10) dans la zone de passage (64) transversale par rapport à l'axe longitudinal de zone de passage (66),
dans lequel en particulier la surface de contact (72) est supérieure d'au moins 50 % à une surface de section transversale du clip (10) dans la zone de passage (64) transversale par rapport à l'axe longitudinal de zone de passage (66), en particulier d'au moins 100 %.

11. Clip médical selon la revendication 9 ou 10, **caractérisé en ce qu'**une saillie de liaison (82) pointant en direction de la au moins une autre partie de clip (30, 32) est réalisée sur une des parties de clip (30, 32) et qu'un logement de saillie de liaison (84), qui reçoit la saillie de liaison (82) à force et/ou par coopération de formes et/ou par liaison de matière, est réalisé sur la au moins une autre partie de clip (30, 32), dans lequel en particulier au moins un dégagement (86) est réalisé sur la saillie de liaison (82) et/ou sur le logement de saillie de liaison (84).

12. Clip médical (10) selon l'une quelconque des revendications précédentes, **caractérisé en ce que** l'élément de précontrainte (24) est réalisé sous la forme d'un ressort hélicoïdal (52) avec au moins un enroulement complet et **en ce qu'**un logement d'enroulement (100) est réalisé sur l'élément de précontrainte (24) dans la zone de la première extrémité (26) et/ou de la deuxième extrémité (28) et **en ce que** le logement d'enroulement (100) est réalisé de manière à pointer en direction de l'enroulement (102) voisin,
dans lequel en particulier le logement d'enroulement (100) est réalisé par un aplatissement (104) ou un évidement plat sur l'élément de précontrainte (24).

13. Procédé pour fabriquer un clip médical (10), en particulier sous la forme d'un clip pour anévrisme (12), dans lequel le clip (10) est réalisé avec un premier bras de serrage (14), un deuxième bras de serrage (16) et un élément de précontrainte (24), de telle sorte que le premier bras de serrage (14) est relié à une première extrémité (26) de l'élément de précontrainte (24), que le deuxième bras de serrage (16) est relié à une deuxième extrémité (28) de l'élément de précontrainte (24) et que le premier bras de serrage (14) et le deuxième bras de serrage (16) dans une position de base du clip (10) sont rapprochés au maximum l'un de l'autre, en particulier de manière à s'appliquer l'un sur l'autre, et à l'encontre de l'action de l'élément de précontrainte (24) peuvent s'éloigner l'un de l'autre à partir de la position de base dans une position ouverte, dans lequel le clip médical (10) est réalisé avec au moins une première partie de clip (30) et au moins une deuxième partie de clip (32), **caractérisé en ce que** le clip médical (10) est réalisé en partie par un procédé de fabrication additive et que la au moins une deuxième partie de clip (32) est formée directement sur la au moins une première partie de clip (30) par un procédé de fabrication additive.

14. Procédé selon la revendication 13, **caractérisé en ce que** la au moins une première partie de clip (30)
a) est réalisée par un processus de formage à froid ou par un procédé de fabrication additive
et/ou
b) est réalisée sous la forme de l'élément de précontrainte (24) et que l'élément de précontrainte (24) lors de la formation de la deuxième partie de clip (32) est retenu par précontrainte, en particulier par torsion du ressort hélicoïdal (52).

15. Utilisation d'un procédé selon la revendication 13 ou 14 pour la fabrication d'un clip médical (10) selon l'une quelconque des revendications 1 à 12.
